(19) Europäisches Patentamt / European Patent Office / Office européen des brevets

(11) **EP 3 188 647 B1**

(12) **EUROPEAN PATENT SPECIFICATION**

(45) Date of publication and mention
of the grant of the patent:
**27.02.2019 Bulletin 2019/09**

(21) Application number: **15767340.1**

(22) Date of filing: **03.09.2015**

(51) Int Cl.:
*A61B 5/00* (2006.01)

(86) International application number:
**PCT/JP2015/004475**

(87) International publication number:
**WO 2016/035344 (10.03.2016 Gazette 2016/10)**

(54) **PHOTOACOUSTIC APPARATUS AND INFORMATION ACQUISITION APPARATUS**

FOTOAKUSTISCHE VORRICHTUNG UND INFORMATIONSERFASSUNGSVORRICHTUNG

APPAREIL PHOTO-ACOUSTIQUE ET APPAREIL D'ACQUISITION D'INFORMATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.09.2014 US 201462046364 P**

(43) Date of publication of application:
**12.07.2017 Bulletin 2017/28**

(73) Proprietor: **Canon Kabushiki Kaisha
Tokyo 146-8501 (JP)**

(72) Inventors:
• **FUKUTANI, Kazuhiko
Tokyo 146-8501 (JP)**
• **KRUGER, Robert A
Oriental, North Carolina 28571 (US)**

(74) Representative: **Houle, Timothy James et al
Canon Europe Ltd
European Patent Department
3 The Square
Stockley Park
Uxbridge, Middlesex UB11 1ET (GB)**

(56) References cited:
**US-A1- 2013 197 344     US-A1- 2014 064 030**

• **KRUGER ROBERT A ET AL: "Dedicated 3D
photoacoustic breast imaging", MEDICAL
PHYSICS, AIP, MELVILLE, NY, US, vol. 40, no. 11,
10 October 2013 (2013-10-10), pages
113301-1-113301-8, XP012178397, ISSN:
0094-2405, DOI: 10.1118/1.4824317 cited in the
application**
• **KRUGER ROBERT ET AL: "Photoacoustic
angiography of the breast", MEDICAL PHYSICS,
AIP, MELVILLE, NY, US, vol. 37, no. 11, 1
November 2010 (2010-11-01), pages 6096-6100,
XP012144778, ISSN: 0094-2405, DOI:
10.1118/1.3497677**

# Description

## Background Art

The present invention relates to a photoacoustic apparatus that receives a photoacoustic wave generated by irradiating an object with light and acquires object information. The present invention also relates to an information acquisition apparatus for acquiring information about an object by using a photoacoustic wave generated by irradiating the object with light.

Optical imaging apparatuses are actively studied in the medical field. Optical imaging apparatuses irradiate an object, such as a living body, with light emitted from a light source, such as a laser, and form an image of information about the inside of the object, which is obtained on the basis of the light with which the object is irradiated. Examples of such an optical imaging technology include photoacoustic imaging (PAI). With photoacoustic imaging, an object is irradiated with pulsed light emitted from a light source; and an acoustic wave (typically, an ultrasonic wave) generated from object tissues (acoustic source), which have absorbed the energy of the pulsed light that has propagated and dispersed in the object, is received. Then, with photoacoustic imaging, imaging (forming of an image) of information about the inside of the object is performed on the basis of the received signal. That is, by using the difference in optical energy absorptance between a part of the object to be observed, such as a tumor, and other tissues, an elastic wave (photoacoustic wave) that is generated when the part of the object instantaneously expands by absorbing the energy of emitted light is received by an acoustic wave receiver. By mathematically processing the received signal, information about the inside of the object, such the generated acoustic pressure distribution and the absorption coefficient distribution, can be acquired. In recent years, by using photoacoustic imaging, preclinical studies of imaging of a blood vessel of a small animal and clinical studies of application of this principle to diagnosis of breast cancer and the like have been actively carried out (see NPL 1).

## Citation List

## Non Patent Literature

NPL 1: "Photoacoustic imaging in biomedicine", M. Xu, L. V. Wang, REVIEW OF SCIENTIFIC INSTURUMENT, 77, 041101, 2006)

## Summary of Invention

## Technical Problem

In a photoacoustic image obtained by using a photoacoustic apparatus, an image of an acoustic source may be buried in image noise. KRUGER ROBERT A ET AL "Dedicated 3D photoacoustic breast imaging", MEDICAL PHYSICS, AIP, MELVILLE, NY, US, vol. 40, no. 11, 10 October 2013 pages 113301-1 - 113301-8, discloses a photoacoustic breast imaging apparatus using spiral scan patterns, where reconstructed images are processed by applying a depth-dependent exponential scaling to compensate for the exponential loss of contrast as a function of the thickness of breast tissue.

## Solution to Problem

The invention is defined by the claims. A photoacoustic apparatus disclosed in the present description includes a light irradiation unit that irradiates an object with light, an acoustic wave receiving unit that receives an acoustic wave generated by irradiating the object with light emitted from the light irradiation unit and that outputs an electric signal, a control unit that controls the light irradiation unit, and a processing unit that processes the electric signal. The control unit controls the light irradiation unit so that a density of a distribution of emission positions in a plurality of times of light emission by the light irradiation unit is higher in an inner part of the distribution of the emission positions than in an outer part of the distribution. The acoustic wave receiving unit outputs a plurality of electric signals corresponding to the plurality of times of light emission by receiving acoustic waves generated by the plurality of times of light emission. The processing unit acquires image data on the basis of the plurality of electric signals.

Further aspects of the present invention will become apparent from the following description of embodiments with reference to the attached drawings.

## Brief Description of Drawings

Fig. 1 is a schematic view illustrating an example of the structure of a photoacoustic apparatus according to the present embodiment.
Fig. 2A illustrates an example of a measurement position and an emission position at a certain light emission timing according to the present embodiment.
Fig. 2B illustrates an example of a measurement position and an emission position at another light emission timing according to the present embodiment.
Fig. 3A illustrates an example of a light amount distribution in an object at a certain light emission timing according to the present embodiment.
Fig. 3B illustrates an example of a light amount distribution in an object at another light emission timing according to the present embodiment.
Fig. 4A is a schematic view illustrating an example of the distribution of measurement positions or emission positions according to a comparative example.
Fig. 4B illustrates an example of the total light amount according to the comparative example.

Fig. 5A is a schematic view illustrating an example of the distribution of the measurement positions or the emission positions according to the present embodiment.

Fig. 5B illustrates an example of the total light amount according to the present embodiment.

Fig. 6 is a schematic view illustrating an example of another distribution of the measurement positions or the emission positions according to the present embodiment.

Fig. 7 is a schematic view illustrating a part of the structure of a photoacoustic apparatus according to an example.

Fig. 8 is a schematic view illustrating an example of the structure of a photoacoustic apparatus according to the example.

Fig. 9 is a schematic view illustrating an example of the structure of the photoacoustic apparatus according to the example.

Fig. 10A illustrates a photoacoustic image acquired by a photoacoustic apparatus according to example 1.

Fig. 10B illustrates a photoacoustic image acquired by a photoacoustic apparatus according to the comparative example.

Fig. 11A is a view for describing an information acquisition apparatus according to the present embodiment.

Fig. 11B is a view for describing the information acquisition apparatus according to the present embodiment.

## Description of Embodiments

A photoacoustic apparatus according to the present embodiment is an apparatus that acquires object information from a received signal of a photoacoustic wave generated by photoacoustic the effect. Examples of the object information (photoacoustic image) that can be acquired from a received signal of a photoacoustic wave include an initial acoustic pressure distribution of the photoacoustic wave, an optical energy absorption density distribution, an absorption coefficient distribution, and a distribution of the concentration of a substance included in the object. Examples of the concentration of a substance include oxygen saturation, oxyhemoglobin concentration, deoxyhemoglobin concentration, and total hemoglobin concentration. The total hemoglobin concentration is the sum of the oxyhemoglobin concentration and the deoxyhemoglobin concentration.

## Structure of Photoacoustic Apparatus

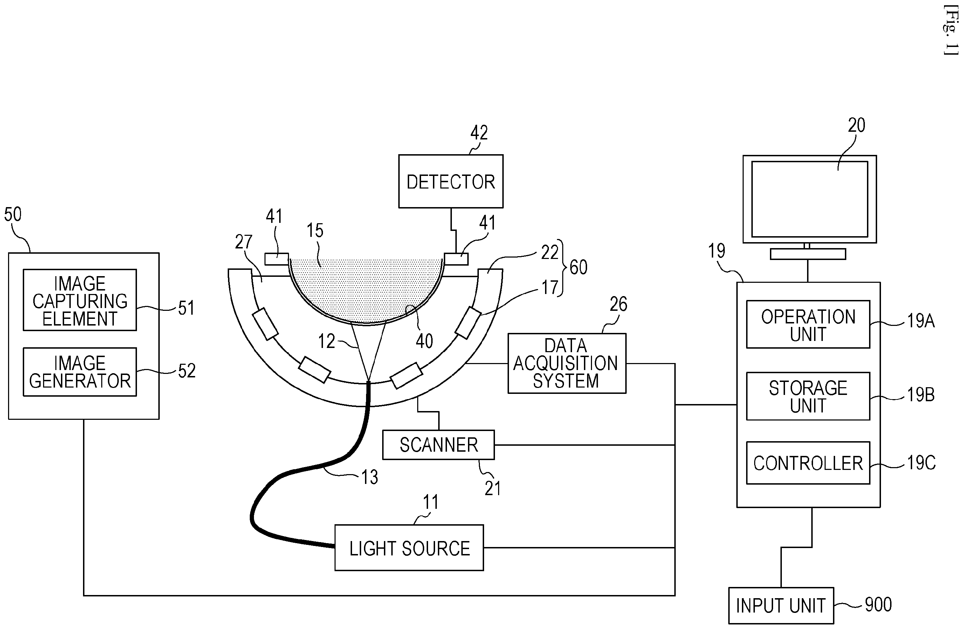

Referring to Fig. 1, the structure of the photoacoustic apparatus according to the present embodiment will be described.

The photoacoustic apparatus according to the present embodiment has a basic hardware structure including a light source 11, an optical system 13, an acoustic wave receiver 60, a scanner 21, an input unit 24, a controller 19C, a data acquisition system 26, and a computer 19. In Fig. 1, a supporter 22 is shown in cross section. The optical system 13 includes light emission ends (not shown) from which light is emitted.

Hereinafter, the elements of the photoacoustic apparatus according to the present embodiment will be described.

## Light Source 11

The light source 11 supplies optical energy to an object and causes the object to generate a photoacoustic wave. In a case where the object is the object, the light source 11 emits light having a specific wavelength that is absorbed by a specific one of the components of the object. The light source may be integrated with the photoacoustic imaging apparatus according to the present embodiment or may be an independent unit. The light source may be a pulsed light source that can generate pulsed light of the order from several nanometers to several hundreds of nanometers as irradiation light. To be specific, in order to efficiently generate a photoacoustic wave, a pulse width in the range of 10 to 100 nanoseconds is used. The light source may be a laser, with which high power can be obtained. Instead of a laser, a light emitting diode or the like can be used. Examples of the laser include a solid-state laser, a gas laser, a fiber laser, a dye laser, a semiconductor laser, and various other lasers. The timing of light emission, the waveform of light, and the intensity of light are controlled by a light source controller. In the present invention, the wavelength of a light source used may be such a wavelength that, in a case where the object is a living body, light can propagate into the living body. To be specific, the wavelength is 500 nm or larger and 1200 nm or smaller.

## Optical System 13

Pulsed light 12 emitted from the light source 11 is guided toward the object while being processed to have a desired light distribution shape by optical components, which are typically lenses, mirrors, and the like. Alternatively, it is also make light propagate through an optical fiber; an optical fiber bundle; or optical waveguides, such as an articulated arm including a lens barrel, a mirror, or the like. Such devices are also regarded as the optical system 13. Other examples of the optical system 13 include a mirror that reflects light, a lens that causes light to converge or diverge or that changes the shape of light, and a diffuser panel that diffuses light. Any of such optical components may be used, as long as an object 15 can be irradiated in a desired shape with the pulsed light 12 emitted from the light source. In view of increasing the diagnosed area of the object, the optical system 13 may expand the light to a certain area. Typically, an emission position from which light is emitted from the optical system 13 is the same as the position of a light emission end of the optical system 13.

If the pulsed light 12 can be emitted desirably from the light source 11, it is not necessary that the photoacoustic apparatus include the optical system 13.

## Object 15

The object 15, which is not included in the photoacoustic apparatus according to the present embodiment, will be described below. The photoacoustic apparatus according to the present embodiment is mainly used to diagnose malignant tumors and blood vessel diseases of a human or an animal and to follow up chemotherapy. Therefore, it is assumed that the object 15 is a breast, a finger, a hand, or a foot of a human or an animal, which is to be diagnosed. A light absorber inside the object may be assumed to be a substance that has a relatively high absorption coefficient in the object 15. For example, if a human body is to be measured, the light absorber corresponds to oxy-hemgoglobin, reduced hemoglobin, or a blood vessel or a newborn blood vessel in which a large amount of such hemoglobin are present. Examples of a light absorber at a surface of the object 15 include melanin. However, by appropriately selecting the wavelength of light, other substances in the human body, such as fat, water, and collagen, may serve as a light absorber.

## Acoustic Wave Receiving Element 17

The acoustic wave receiver 60 as an acoustic wave receiving unit includes a plurality of acoustic wave receiving elements 17 and the supporter 22. Each acoustic wave receiving element 17 receives an acoustic wave and converts the acoustic wave into an analog electric signal. Each acoustic wave receiving element 17 may be any device that can detect an acoustic wave, such as a piezoelectric transducer, a transducer using optical resonance, or a transducer using a change in capacity. In the present embodiment, the plurality of acoustic wave receiving elements 17 are disposed. By using such a multidimensional-array element, an acoustic wave can be simultaneously received at a plurality of positions. Therefore, a receiving time can be reduced, and the influence of vibration of the object 15 or the like can be reduced.

## Supporter 22

The plurality of acoustic wave receiving elements 17 are disposed along the supporter 22. The supporter 22 may be configured so that the plurality of acoustic wave receiving elements 17 are disposed on a closed curved surface surrounding the object 15. However, if the object is a human body or the like and it is difficult to dispose the plurality of acoustic wave receiving elements 17 on any closed curved surface that surrounds the object, the plurality of acoustic wave receiving elements 17 may be disposed on a surface of the hemispherical supporter 22 as shown in Fig. 1. If it is difficult to dispose the plurality of acoustic wave receiving elements 17 on a hemispherical supporter, the plurality of acoustic wave receiving elements 17 may be disposed on a planar supporter.

In general, each of the plurality of acoustic wave receiving elements 17 has the highest receiving sensitivity in a direction perpendicular to the receiving face (surface) thereof. By making the directional axes of the plurality of acoustic wave receiving elements 17 converge toward the center of the curvature of the hemispherical shape, a high-resolution region 23 that can be visualized with high precision is formed.

For example, the high-resolution region 23 is considered to be a substantially hemispherical region that is centered at the center of curvature for obtaining the highest resolution and that has a radius r represented by expression (1).

[Math.1]

$$r = \frac{r_0}{\phi_d} \cdot R \qquad (1)$$

R is the lower limit resolution of the high-resolution region 23, $r_0$ is the radius of the hemispherical supporter 22, and $\Phi_d$ is the diameter of the acoustic wave receiving element 17. R may be a resolution that is, for example, a half of the highest resolution that is obtained at the center of curvature.

A case where the high-resolution region 23 has a substantially hemispherical shape centered at the center of curvature of the supporter 22 is considered. In this case, the computer 19 can estimate the range of the high-resolution region 23 at each measurement position in accordance with expression (1).

In the present invention, the disposition of the plurality of acoustic wave receiving elements 17 is not limited to the example of the hemispherical shape shown in Fig. 1. The plurality of acoustic wave receiving elements 17 may be disposed in any way, as long as the directional axes of the plurality of acoustic wave receiving elements 17 converge in a predetermined region and the predetermined high-resolution region 23 can be formed. That is, the plurality of acoustic wave receiving elements 17 may be disposed along a curved surface so as to form a predetermined region so that the predetermined high-resolution region 23 can be formed. Examples of a curved surface in the present description include a perfect sphere or a hemisphere having an opening. Examples of a curved surface further include a surface having protrusions and recesses to a degree that the surface can be regarded as a sphere and a surface of an ellipsoid (a three-dimensional quadratic surface that is a three-dimensional extension of an ellipse) that can be regarded as a sphere.

When the plurality of acoustic wave receiving elements are disposed along a supporter having the shape of a sphere cut along an arbitrary sectional plane, the directional axes converge most at the center of the shape of the supporter. The hemispherical supporter 22 described in the present embodiment is also an example of a supporter having the shape of a sphere cut along an arbitrary sectional plane. In the present description, the shape of a sphere cut along an arbitrary sectional plane will be referred to as a "shape based on a sphere". A plurality of acoustic wave receiving elements that are disposed on a supporter having the shape based on a sphere are considered to be disposed on a sphere.

The supporter 22 has an irradiation hole, for guiding the pulsed light 12, in a bottom surface thereof. Light guided by the optical system 13 is emitted through the irradiation hole toward the object 15. The optical system 13 and the irradiation opening may be considered to constitute an optical system according to the present invention.

**Data Acquisition System 26**

The photoacoustic apparatus according to the present embodiment includes the data acquisition system 26, which amplifies an electric signal obtained by the acoustic wave receiving elements 17 and which converts the electric signal from an analog signal into a digital signal. Typically, the data acquisition system 26 includes an amplifier, an A/D converter, a FPGA (field programmable gate array) chip, and the like. In a case where a plurality of received signals can be obtained from a probe, the plurality of signals may be simultaneously processed. By doing so, a time required to form an image can be reduced. In the present description, the term "received signal" refers not only to an analog signal acquired from the acoustic wave receiving elements 17 but also to a digital signal generated by subsequently performing A/D conversion of the analog signal. The received signal may be referred to as a "photoacoustic signal".

**Computer 19**

The computer 19, which corresponds to a processing unit, includes an operation unit 19A, a storage unit 19B, and the controller 19C as a control unit. The computer 19 can perform predetermined processing on electric signals output from the plurality of acoustic wave receiving elements 17. The computer 19 can control the operation of each element of the photoacoustic apparatus.

Typically, the operation unit 19A includes elements, such as a CPU, a GPU (graphics processing unit), and an A/D converter; and circuits, such as a FPGA and an ASIC. The operation unit 19A may include not only a single element or a single circuit but may include a plurality of elements and a plurality of circuits. Each processing performed by the computer 19 may be performed by any of the element or circuit.

Typically, the storage unit 19B includes a storage medium, such as a ROM, a RAM, or a hard disk. The storage unit 19B may include not only a single storage medium but may include a plurality of storage media.

Typically, the controller 19C includes a CPU.

The computer 19 may be configured to perform pipeline processing of a plurality of signals simultaneously. By doing so, a time required to acquire object information can be reduced.

The operation unit 19A acquires object information in each voxel by preforming image reconstruction processing on received signal data stored in the storage unit 19B. Examples of image reconstruction processing include time domain backprojection and Fourier domain backprojection, and phase addition processing, which are generally used in the tomography technology. An image reconstruction method such as inverse problem analysis using an iterative processing may be used. By using a probe including an acoustic lens or the like and having a receiving focus function, object information may be visualized without performing image reconstruction. The operation unit 19A, which performs image reconstruction processing, generally includes a GPU, which has a high-performance calculation function and a graphic display function. Thus, a time required to perform image reconstruction processing or to form a display image can be reduced.

The controller 19C reads program codes stored in the storage unit 19B and controls various parts of the photoacoustic apparatus. For example, the controller 19C controls driving of the scanner 21, the light emission timing of the light source 11, and the like. By controlling the scanner 21, the controller 19C can control parameters related to movement of the supporter 22, such as the movement velocity, the movement direction, and the movement range of the supporter 22. The movement range may be changed in accordance with the size of the object. For example, extra measurement time can be reduced by controlling movement of the supporter 22 in a small movement range if the object is small and by controlling the movement in a large movement range if the object is large. Note that the storage unit in which the program is stored is a non-transitory recording medium.

The functions of the computer 19 may be performed by different hardware devices. The acoustic wave receiver 60, the data acquisition system 26, and the computer 19 may be integrated in a single hardware device. Some of these elements may be integrated in a single hardware device. For example, the acoustic wave receiver 60 and the data acquisition system 26 may be integrated in a single hardware device.

**Display Unit 20**

A display unit 20 is a device for displaying image data output from the computer 19. Typically, a liquid crystal display is used as the display unit 20. The display unit 20 may be independent from the photoacoustic apparatus.

**Scanner 21**

The scanner 21, which corresponds to a moving unit, includes a driving unit, such as a motor, and mechanical components for transmitting a driving force of the motor.

In accordance with position control information from the controller 19C, the scanner 21 moves the measurement position of the supporter 22 or the emission position of light from the optical system 13 relative to the object 15. By repeating acquisition of received signal data while moving the emission position and the measurement position relative to the object 15, received signal data for obtaining object information for a targeted wide area can be acquired.

In synchronism with irradiation control of the pulsed light 12 by the controller 19C, the scanner 21 outputs position information about the supporter 22 or the optical system 13 during light irradiation, that is, during receiving of a photoacoustic wave, to the computer 19.

The scanner 21 may be capable of moving at least one of the optical system 13, the acoustic wave receiving elements 17, and the object 15.

The scanner 21 may be a step-and-repeat scanner that repeats moving and stopping or a continuous movement scanner that continuously moves. However, with a continuous movement scanner that continuously moves, the total movement time can be reduced and a load on a subject can be reduced. Moreover, by performing continuous movement with a small change in the acceleration of movement, the influence of shaking of the apparatus can be reduced.

The scanner 21 may move the optical system 13 together with the supporter 22 in order to move the irradiation position of the pulsed light 12 emitted from the light source 11. That is, the supporter 22 and the optical system 13 may be moved in synchronism with each other. By doing so, the relationship between the acoustic wave receiving position and the light irradiation position is maintained constant, so that more uniform object information can be acquired. In the present embodiment, a unit that moves the relative positions of the optical system 13 and the object 15 will be referred to as an emission position moving unit, and a unit that moves the relative positions of the acoustic wave receiver 60 and the object 15 will be referred to as a measurement position moving unit.

In the case where the object is a human body, the irradiation area of the object over which irradiation can be performed is limited by the standard of American National Standards Institute (ANSI). Therefore, in order to increase the amount of light that propagates into the object 15, the irradiation intensity and the irradiation area may be increased, but the irradiation area is limited in view of the cost of the light source. Moreover, due to the directivity of a receiving element, the utilization efficiency of the amount of light is low if a region having a low receiving sensitivity is irradiated with light. Therefore, it is not efficient to irradiate the entirety of a large object with light. That is, light may be moved together with the acoustic wave receiver, because it is efficient to irradiate with light only a region in which the sensitivity of the acoustic wave receiver is high.

**Input Unit 24**

The input unit 24 may include a mouse, a keyboard, or the like, which can be operated by a user. The display unit 20 may include a touch panel, and the display unit 20 may be used as the input unit 24. Examples of operations that a user can perform by using the input unit 24 include setting of parameters related to imaging; instruction for starting imaging; setting of observation parameters, such as the range and the shape of a region of interest; and other image processing operations related to an image.

The elements of the photoacoustic apparatus may be configured as independent devices or a single integrated device. Alternatively, at least some of the elements of the photoacoustic apparatus may be configured as a single integrated device.

**Acoustic Matching Member 27**

A space between the object 15 and the supporter 22, which is a propagation path of a photoacoustic wave, may be filled with an acoustic matching member 27, such as water or a gel for ultrasonic measurement, so that an air gap may not be formed in the space. The acoustic matching member 27, which also serves as a propagation path of the pulsed light 12, may be a medium that is transparent with respect to the pulsed light 12. For example, water is used as the acoustic matching member 27.

**Holding Unit 40**

A holding unit 40 is used to hold the shape of the object 15 during measurement. By holding the object 15 using the holding unit 40, movement of the object can be suppressed and the position of the object 15 can be retained in the holding unit 40. PET-G or the like can be used as the material of the holding unit 40.

The holding unit 40 may be made of a material having a hardness with which the holding unit 40 can hold the object 15. The holding unit 40 may be made of a material that transmits light used for measurement. The holding unit 40 may be made of a material having substantially the same impedance as the object 15. When the object 15 is an object having a curved surface, such as a breast, the holding unit 40 may have a concave shape. In this case, the object 15 can be inserted into the concave portion of the holding unit 40.

## Image Capturing Device 50

An image capturing device 50 generates image data of the object and outputs the generated image data to the computer 19. The image capturing device 50 includes an image capturing element 51 and an image generator 52. The image generator 52 generates image data of the object by analyzing a signal output from the image capturing element 51, and the generated image data is stored in the storage unit 19B.

For example, an optical image capturing element, such as a CCD sensor or a CMOS sensor, may be used as the image capturing element 51. A transducer that can transmit and receive an acoustic wave, such as a piezoelectric element or CMUT, may be used as the image capturing element 51. Some of the plurality of acoustic wave receiving elements 17 may be used as the image capturing element 51. Any element may be used as the image capturing element, as long as the image generator 52 can generate an image of the object on the basis of a signal output from the image capturing element 51.

The image generator 52 includes elements, such as a CPU, a GPU, and an A/D converter; and circuits, such as a FPGA and an ASIC. The computer 19 may also function as the image generator 52. That is, the operation unit 19A in the computer 19 may be used as the image generator 52.

## Operation of Photoacoustic Apparatus

Next, the operation of the photoacoustic apparatus according to the present embodiment will be described. The controller 19C drives the scanner 21 in accordance with instruction from the computer 19 and moves the acoustic wave receiver 60 (supporter 22) along a desired movement path. At this time, at a certain timing, the pulsed light 12 emitted from the light source 11 is guided by the optical system 13; and the object 15, such as a living body, is irradiated with the pulsed light 12. At the timing at which the object 15 is irradiated with the pulsed light 12, the pulsed light 12 reaches the entirety of the inside of the object 15 substantially simultaneously. When a part of the energy of the pulsed light 12 that has propagated through the inside of the object 15 is absorbed by a light absorber (which is to serve as an acoustic source), such as a blood vessel in which a large amount of hemoglobin is included, a photoacoustic wave (typically, an ultrasonic wave) is generated due to thermal expansion of the light absorber. The plurality of acoustic wave receiving elements 17 receive the photoacoustic wave and converts the photoacoustic wave into an electric signal.

Typically, the propagation velocity of the photoacoustic wave is higher than the velocity with which the scanner 21 moves the acoustic wave receiver 60. Therefore, the photoacoustic wave is received at positions at which the plurality of acoustic wave receiving elements 17 are located at the emission timing of the pulsed light 12. Thus, movement of the acoustic wave receiver 60 during a period from the time at which the pulsed light 12 is emitted toward the object 15 to the time at which the plurality of acoustic wave receiving elements 17 receive the photoacoustic wave may be neglected. Therefore, in the present embodiment, the light emission timing of the pulsed light 12 is regarded as the timing at which the photoacoustic wave is received. The position of the supporter 22 relative to the object 15 at the light emission timing of the pulsed light 12 will be referred to as the "measurement position of the supporter 22 (measurement position of the acoustic wave receiver 60)".

In the present embodiment, the position of the center of the supporter 22 is regarded as the position of the supporter 22. The position of the optical system 13 relative to the object 15 at the light emission timing of the pulsed light 12 will be referred to as the "emission position of light from the optical system 13".

In the present embodiment, the supporter 22 includes the plurality of acoustic wave receiving elements 17. Therefore, it is possible to identify the positions of the plurality of acoustic wave receiving elements 17 at each light emission timing from the measurement position of the supporter 22 at each light emission timing. In the present embodiment, the scanner 21 moves the supporter 22 and the optical system 13 while maintaining the positional relationship between the supporter 22 and the optical system 13. Therefore, the positional relationship between the emission position from which light is emitted from the optical system 13 and the measurement position of the supporter 22 at each light emission timing is constant. Hereinafter, "the measurement position of the supporter 22" may be simply referred to as the "measurement position". The "emission position of light from the optical system 13" may be simply referred to as the "emission position".

Next, the controller 19C drives the scanner 21 in accordance with instruction from the computer 19 to move the optical system 13 and the supporter 22. At this time, the pulsed light 12 is emitted at another timing, and the plurality of acoustic wave receiving elements 17 receive a newly generated photoacoustic wave. In this way, the photoacoustic wave can be received at an emission position and a measurement position that differ from the emission position and the measurement position at the previous timing. In the present embodiment, because the light source 11 generates the pulsed light 12 with a predetermined cyclic frequency, the emission position and the measurement position can be controlled by controlling the scanner 21. In this case, the light source 11, the optical system 13, and the scanner 21 constitute a light irradiation unit; and the controller 19C can control the emission position by controlling the scanner 21 as the light irradiation unit.

The controller 19C may change the emission timing of the light source 11. In this case, the controller 19C controls the emission position and the measurement position by controlling the light emission timing of the light source 11 and driving of the scanner 21. In this case, the light source 11, the optical system 13, and the scanner 21 constitute a light irradiation unit; and the controller 19C can control the emission position by controlling the light source 11 and the scanner 21 as the light irradiation unit.

The optical system 13 may include a plurality of light emission ends, and the controller 19C may control the emission position by controlling from which light emission end light is emitted. In this case, the light source 11 and the optical system 13 constitute a light irradiation unit, and the controller 19C controls the emission position by controlling the optical system 13 as the light irradiation unit. For example, by including an optical filter that reduces light and or a beam splitter that splits a light, the optical system 13 can change the light emission end from which light is emitted. Because the power of the light source 11 is limited, if light is emitted from a large number of emission ends at one time of light emission, the intensity of light emitted from each emission end becomes low. In this case, there are some positions in the object to which only a small amount of light reaches at any light emission timing. It may be difficult to form an image of such a position with high precision. Therefore, in one time of light emission from the light source 11, the light may be split to some of the plurality of emission ends, and the object may be irradiated with light emitted from the some of the emission ends. The emission ends to which light is split may differ between the first light emission timing (first light emission) and the second light emission timing (second light emission) so that light can be emitted from all of set emission positions through a plurality of times of light emission.

Next, the data acquisition system (DAS) 26 performs amplification and digital conversion of an electric signal output from the plurality of acoustic wave receiving elements 17.

Next, the computer 19 sets a region of interest, whose image based on object information is to be displayed. The computer 19 may set a region that a user has specified by using the input unit 24 as a region of interest. Alternatively, the computer 19 may set a region specified by a user in an image obtained by another image forming apparatus (for example, the image capturing device 50) as a region of interest. Further alternatively, the computer 19 may set a region of interest by analyzing an image obtained by another image forming apparatus (for example, the image capturing device 50). Further alternatively, the computer 19 may be set a region of interest by reading information stored beforehand in the storage unit 19B. For example, when setting the entire region of the object as a region of interest, a region including the entire region in the holding unit 40 may be set beforehand as a region of interest.

Next, the computer 19 acquires object information in the region of interest, such as an initial acoustic pressure distribution and an absorption coefficient distribution, by performing predetermined processing, such as image reconstruction, on a digital signal output from the data acquisition system 26. For example, by performing image reconstruction processing described in U.S. Patent No. 5713356, object information for each voxel can be acquired from electric signals obtained at a plurality of light emission timings. Typically, the computer 19 acquires object information for each voxel by superposing a plurality of electric signals. If the number of electric signals used for calculation differs between the voxels, the superposed value may be divided by the number of signals used for calculation. By doing so, variation of object information due to variation in the number of signals can be reduced. Note that, if the number of electric signals used for calculation does not differ between voxels, the superposed value may be used as it is.

Next, the computer 19 generates image data to be displayed on the display unit 20 on the basis of the acquired object information. Then, the computer 19 causes the display unit 20 to display a photoacoustic image based on the generated image data.

A user, such as a doctor, can use the photoacoustic image of the object information, which is displayed on the display unit 20 as described above, for diagnosis and the like.

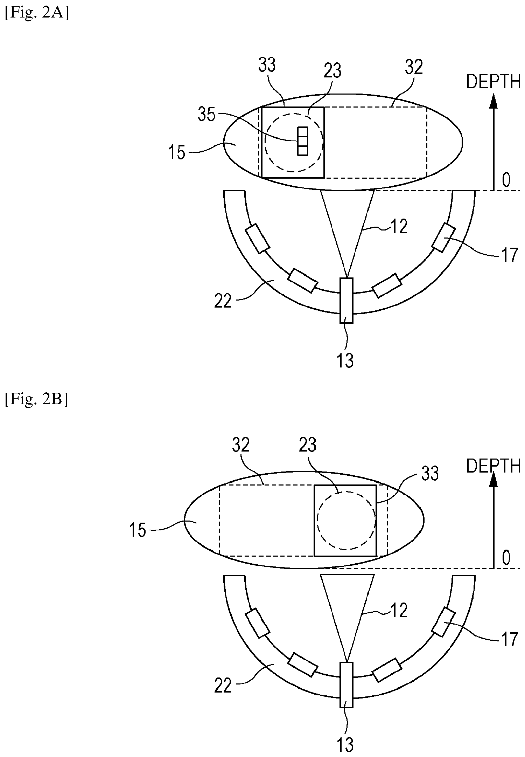

For example, Figs. 2A and 2B illustrate an example of the positions of the optical system 13 and the supporter 22 at certain light emission timings A and B. That is, Figs. 2A and 2B illustrate the emission position and the measurement position at the light emission timings A and B. In each of Figs. 2A and 2B, the direction indicated by an arrow (direction in which the pulsed light 12 is emitted) is the depth direction of the object 15. A photoacoustic image of a region including a region of interest 32 is to be finally displayed on the display unit 20.

As illustrated in Figs. 2A and 2B, the acoustic wave receiving elements 17, which are disposed on the hemispherical supporter 22, form the spherical high-resolution region 23 in the vicinity of the center of curvature of the hemisphere. Here, the high-resolution region 23 is a region in which an image having a resolution up to a half of the maximum resolution can be obtained.

In the present embodiment, the computer 19 sets the high-resolution region 23 or a region including the high-resolution region 23 as an image reconstruction region 33 to be imaged. The computer 19 sets the image reconstruction region 33 corresponding to each light emission timing so that the region of interest 32 is filled with the image reconstruction region 33 corresponding to each light emission timing. In the present embodiment, an example in which the computer 19 performs image reconstruction processing at each light emission timing will be described. However, the computer 19 may perform image reconstruction processing on electric signals obtained at a plurality of light emission timings.

In the present embodiment, the computer 19 sets the region of interest 32 and the image reconstruction region 33 for each light emission timing, and divides the inside thereof into voxels 35. In Fig. 2A, only some of the voxels 35 are shown for convenience.

In the present embodiment, because object information in a three-dimensional space region is to be acquired, the minimum region to be reconstructed is referred to as a "voxel". However, object information in a two-dimensional space region may be acquired. In this case, the minimum region to be reconstructed is referred to as a "pixel".

Alternatively, a user may specify any appropriate region by using the input unit 24, and the computer 19 may set the region specified by the user as the region of interest 32 or the image reconstruction region 33. Further alternatively, the region of interest 32 or the image reconstruction region 33 may be set beforehand.

Hereinafter, the distribution of emission positions or the measurement position according to the present embodiment will be described. In the present embodiment, because the positional relationship between the emission position and the measurement position are constant as described above, the distributions of the emission position and the measurement position are similar to each other.

**Distribution of Emission Positions**

In photoacoustic apparatuses, the acoustic pressure of the generated photoacoustic wave changes in accordance with the light amount distribution in an object. Typically, the generated acoustic pressure of a photoacoustic wave is proportional to the amount of light. That is, even when the object is a blood vessel (blood) having the same absorption coefficient, the light amount distribution in the object is not uniform depending on the light emission method and the shape of the object, and therefore the acoustic pressure generated in the object varies considerably. If the generated acoustic pressure of a photoacoustic wave is low, the intensity of the received signal of the photoacoustic wave is also low. Therefore, in a photoacoustic image obtained by performing image reconstruction processing on the received signal, there may exit a region in which the generated acoustic pressure is not represented by being buried in image noise. That is, regarding a region in which the generated acoustic pressure is low, there may exist a region in which the image noise level is not at a level with which the generated acoustic pressure can be represented.

Therefore, in the present embodiment, the emission position is set so that the total amount of light due to light emission at a plurality of light emission timings corresponding to each voxel in the region of interest 32 is larger than or equal to a threshold. That is, the controller 19C controls the scanner 21 so that, when the region of interest 32 is divided into the voxels, the distribution of emission positions is such that the total amount of light in each voxel by a plurality of times of light emission is larger than or equal to the threshold.

By making the emission position to have such a distribution, the generated acoustic pressure of a photoacoustic wave generated in a voxel in which an acoustic source is present can be made higher than or equal to a predetermined level. Therefore, burying the image of the acoustic source in image noise can be suppressed. Hereinafter, the distribution of emission positions according to the present embodiment will be described. In describing the present embodiment, it is assumed that the light source 11 generates the pulsed light 12 with a constant intensity at each light emission timing.

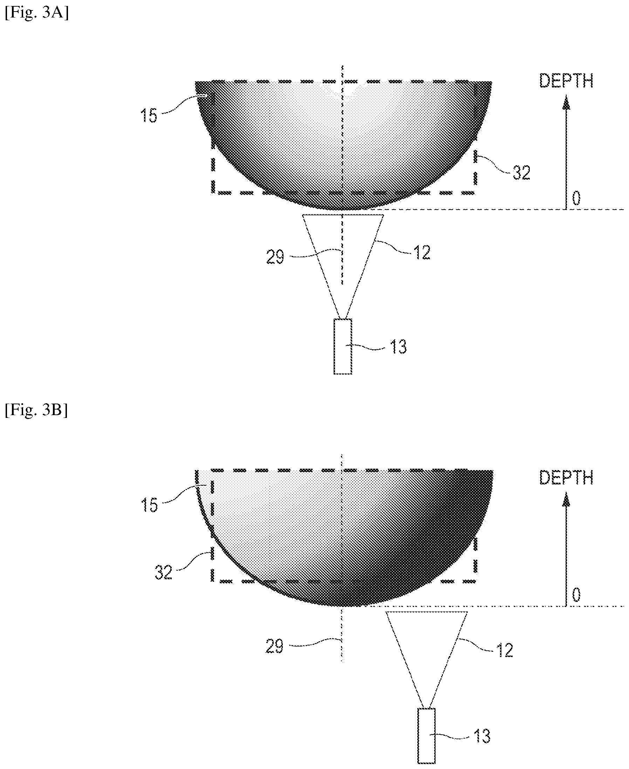

Figs. 3A and 3B illustrate an example of the distribution of the amount of the pulsed light 12 in the object 15 at certain light emission timings A and B. In each of the figures, the black region is a region in which the amount of light is high, and the white region is a region in which the amount of light is low. In each of the figures, the direction indicted by an arrow (the direction in which the pulsed light 12 is emitted) is the depth direction of the object 15.

By changing the emission position between a plurality of timings, the light irradiation position at which the object 15 is irradiated with light is changed. As a result, the amount of light in the object voxels at the light emission timings, that is, the light amount distribution in the object 15 differs as shown in Figs. 3A and 3B. In particular, when the object 15 is hemispherical, the amount of light is relatively low in a deep part of the object 15, because the amount of light is reduced due to diffusion decay. That is, as can be understood from Figs. 3A and 3B, the amount of light is high at the irradiation position of the pulsed light 12 and the amount of light gradually decreases as a position becomes farther from the irradiation position. The light amount distribution in the object 15 at each light emission timing can be acquired by the computer 19 on the basis of, for example, the light emission pattern at each light emission timing, the optical constants of the object 15, information about the coordinates of the surface of the object 15, an the like.

By calculating the sum of the amounts of light in an object voxel at the light emission timings, the total light amount in each voxel can be obtained. The total light amount in each voxel differs depending on the distribution of the emission positions.

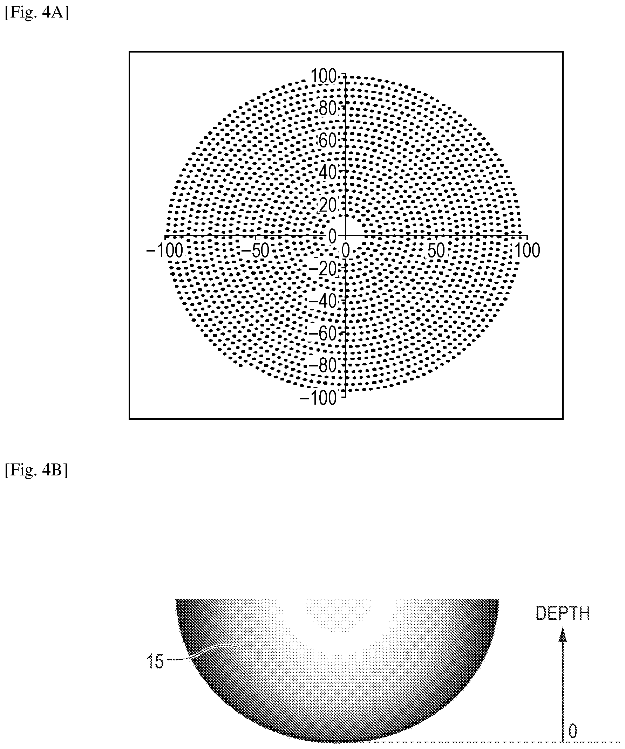

For example, Fig. 4A illustrates the distribution of emission positions according to a comparative example, which is described in NPL 2: "Dedicated 3D Photoacoustic Breast imaging", Robert A. Kruger, Cherie M. Kuzmiak, Richard B. Lam, Daniel R. Reinecke, Stephen P. Del Rio, and Doreen Steed, Medical Physics 40, 113301 (2013). In Fig. 4A, the emission position at each light emission timing is plotted with a dot. NPL 2 proposes a method for uniformly reducing image noise due to reconstruction artifacts, which are generated by image reconstruction, by moving an acoustic wave receiver, on which a plurality of acoustic wave receiving elements are disposed on a hemispherical surface, with an equal distance. In NPL 2, because an optical system is scanned while maintaining the positional relationship between the optical system and the acoustic wave receiver, the density of the distribution of emission positions is uniform as shown in Fig. 4A. Fig. 4B illustrates the total light amount in the voxels in the case where the density of the distribution of emission positions is uniform as shown in Fig. 4A. As can be understood from Fig. 4B, at the same depth in the object 15, the total light amount in voxels in a central part of the object 15 is smaller than the total light amount in voxels in a peripheral part of the object 15.

As described above, in a region in which the amount of light is low, the image of an acoustic source may be buried in image noise. In order to increase the total light amount in voxels in a central part of the object 15 by using the method described in NPL 2, for making the density of the distribution of emission positions be uniform, it is necessary to also perform additional measurement (light irradiation) of voxels in a peripheral part of the object 15, in which the total light amount is sufficiently high. Therefore, in order to make image noise included in each voxel to a desired noise level by using the method described in NPL 2, a long measurement time is needed.

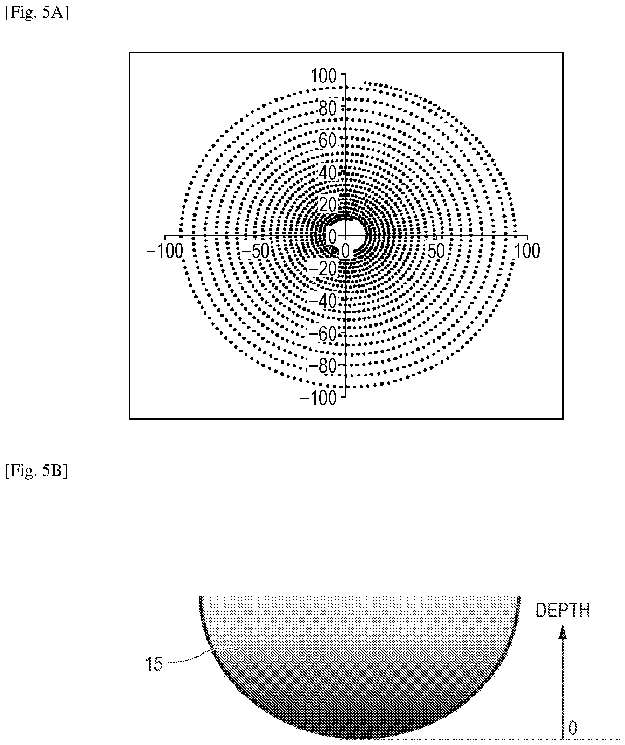

In contrast, in the present embodiment, in order to increase the total light amount in voxels in a central part of the object 15, the distribution of emission positions shown in Fig. 5A is set. That is, the density of the distribution of emission positions is changed, and the distribution of emission positions is set so that the total light amount due to light emission at a plurality of light emission timings corresponding to each voxel in the region of interest 32 is larger than or equal to a threshold.

In the present embodiment, the density of the distribution of emission positions is not uniform, and the density is higher in an inner part of the distribution of the emission positions than in an outer part of the distribution. In the present embodiment, the scanner 21 moves the optical system 13 so that the distance between each emission position and an adjacent emission position changes. In Fig. 5A, the scanner 21 moves the optical system 13 so that the distance between adjacent emission positions decreases from an outer part toward an inner part of a spiral movement path.

By using the distribution of emission positions shown in Fig. 5A, the total light amount in the voxels shown in Fig. 5B can be obtained. In Fig. 5B, the total light amount is substantially uniform at the same depth of the object 15. By using the nonuniform distribution of the emission position, light can be selectively emitted toward voxels in which the total light amount is smaller than a predetermined threshold. Therefore, it is possible to reduce the time required for measurement that is performed to make the total light amount in each voxel be larger than or equal to a predetermined threshold.

In the present invention, as long as the total light amount in each voxel is larger than or equal to a predetermined threshold, the total light amount may be nonuniform at the same depth of the object 15.

As heretofore described, with the present embodiment, the total amount of light with which each voxel is irradiated can be made larger than or equal to a predetermined threshold while reducing the measurement time. Therefore, the generated acoustic pressure of a photoacoustic wave generated in each voxel can be made higher than or equal to a predetermined level, and thereby burying of image of an acoustic source in image noise can be suppressed.

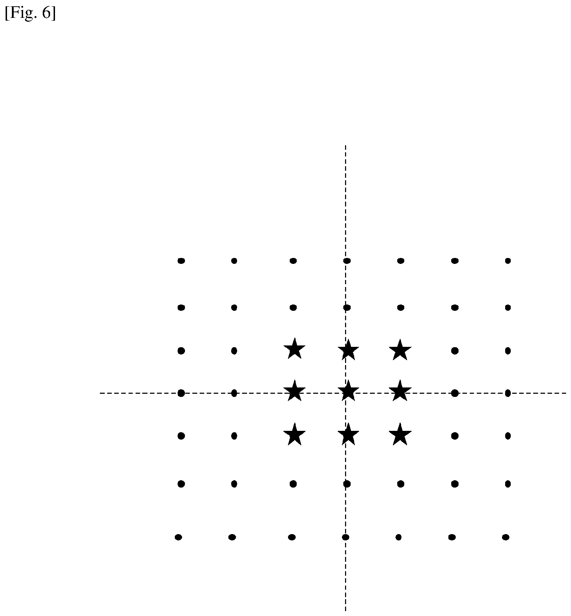

The total light amount in each voxel may be made larger than or equal to a predetermined threshold by emitting light a larger number of times at the positions shown by asterisks in Fig. 6 than at other positions. That is, the number of times light is emitted at emission positions from which light can be emitted toward a central part of the object 15 may be made larger than the number of times light is emitted at other emission positions. To be specific, when the controller 19C moves the optical system 13 to a certain emission position, the controller 19C stops the movement of the optical system 13 at the emission position and causes the light source 11 to emit light a plurality of times at the same emission position. Alternatively, the controller 19C may move the optical system 13 continuously to move the optical system 13 to a previous emission position and causes the light source 11 to emit light at the emission position. By doing so, the object 15 can be irradiated with light a plurality of times at the same emission position.

Also in this case, as shown in Fig. 5B, the total light amount can be made substantially uniform at the same depth of the object 15. Making the number of times of light emission differ between emission positions is included in making the density of the distribution of emission positions differ. That is, in both of the cases shown in Fig. 5A and Fig. 6, the number of emission positions per unit area or per unit volume (distribution density) is not uniform but differs.

Here, a threshold of the amount of light is an apparatus-specific value and differs between the voxels. To be specific, if an object to be imaged is a blood vessel, it is assumed that a substance having the same absorption coefficient as blood exists in the object, simulation of light emission and various measurement positions is performed, and the threshold is determined as a total light amount with which the object can be visually recognized. That is, the threshold is a value that is determined with consideration of apparatus characteristics and the object characteristics. However, if it is difficult to perform simulation, an empirical value may be used. When the threshold of the total light amount is determined, the computer 19 can determine the distribution of emission positions so that the total light amount in each voxel in the region of interest 32 is larger than or equal to the threshold.

If shaking of the acoustic matching member 27 occurs due to movement of the optical system 13 and the supporter 22, it may happen that the space between the object 15 and the acoustic wave receiving elements 17 may not be filled with the acoustic matching member 27. In this case, a photoacoustic wave generated in the object 15 may not be able to reach the acoustic wave receiving elements 17 with a sufficient intensity. For this reason, the scanner 21 moves the optical system 13 and the supporter 22 along a spiral movement path during a plurality of light emission timings. By moving the optical system 13 and the supporter 22 with a small change in acceleration in this way, shaking of the acoustic matching member 27 can be suppressed. The movement path need not be spiral, a long as the optical system 13 and the supporter 22 can be moved with a small change in acceleration so that shaking of the acoustic matching member 27 can be suppressed.

**Distribution of Measurement Positions**

In the present embodiment, the distribution of measurement positions can be set so that burying of the image of an acoustic source in image noise can be suppressed. Hereinafter, the distribution of measurement positions according to the present embodiment will be described.

As described above, if the total light amount is as shown in Fig. 4B, because the generated acoustic pressure of photoacoustic wave is low in the central part of the object 15 as described above, an image of an acoustic source may be buried in image noise due to system noise. Moreover, regarding photoacoustic apparatuses, there exists a factor that influences image noise of a reconstructed image, other than the total light amount in each voxel. For example, image reconstruction artifacts, which are generated because the acoustic source cannot be surrounded by the acoustic wave receiving elements 17 from all directions, may cause image noise. That is, image noise due to image reconstruction artifacts is likely to occur in a region other than the high-resolution region 23. Moreover, because a photoacoustic wave decays as it propagates and a received signal of the photoacoustic wave decreases, the image of an acoustic source may be buried in image noise due to system noise.

In the present embodiment, as shown in Figs. 2A and 2B, the position of the high-resolution region 23 is uniquely determined in accordance with the measurement position of the supporter 22. The plurality of acoustic wave receiving elements 17, which are disposed on the supporter 22, can each receive a photoacoustic wave generated in the high-resolution region 23 with high sensitivity.

Therefore, in the present embodiment, the distribution of measurement positions is set so that image noise included in a reconstructed image is lower than or equal to a predetermined threshold. To be specific, as shown in Fig. 5A, the density of the distribution of measurement positions is set so that the density is higher in an inner part of the distribution of measurement positions than in an outer part of the distribution. In the present embodiment, the scanner 21 moves the supporter 22 so that the distance between each measurement position and an adjacent measurement position changes. In the case of the spiral movement path, the scanner 21 moves the supporter 22 so that the distance between adjacent measurement positions decreases from an outer part toward an inner part of the spiral path.

By receiving the acoustic wave at various positions in this way, image noise due to reconstruction artifacts in a photoacoustic image after image reconstruction can be reduced. Moreover, by increasing the number of times of measurement when the high-resolution region 23 is located in a central part of the object 15, image noise due to system noise included in voxels near the center of the object 15 can be reduced.

Here, a threshold of image noise is an apparatus-specific value and differs between the voxels. To be specific, if an object to be imaged is a blood vessel, it is assumed that a substance having the same absorption coefficient as blood exists in the object, simulation of light emission and various measurement positions is performed, and the threshold is an image noise level with which the object can be visually recognized. That is, the threshold is a value that is determined with consideration of the apparatus characteristics of and the object characteristics. However, if it is difficult to perform simulation, an empirical value may be used. When the value of image noise is determined, the computer 19 can determine the distribution of the measurement positions so that the image noise in each voxels in the region of interest 32 is lower than or equal to the threshold.

Image noise included in each voxel may be made lower than or equal to a predetermined threshold by making the number of times of measurement be larger at the positions shown by asterisks in Fig. 6 than at other positions. That is, the number of times of measurement at measurement positions with which the high-resolution region 23 is located in a central part of the object 15 may be made larger than the number of times of measurement at other measurement positions. To be specific, when the controller 19C moves the supporter 22 to a certain measurement position, the controller 19C stops the movement of the supporter 22 at the measurement position and causes the supporter 22 to emit light a plurality of times at the measurement position. Alternatively, the controller 19C may move the supporter 22 continuously to move the supporter 22 to a previous measurement position and causes the light source 11 to emit light at the measurement position. By doing so, photoacoustic waves can be received at the same measurement position.

Making the number of times of measurement differ in accordance with measurement positions is included in making the density of the distribution of measurement positions differ. That is, in both of the cases of Fig. 5A and Fig. 6, the number the measurement positions per unit area or per unit volume (distribution density) is not uniform but differs.

It is not necessary that both the density of distribution of the emission positions and the density of the distribution of measurement positions be uniform in order to make image noise included in each voxel in the region of interest 32 be lower than or equal to a threshold. In this case, the scanner 21 may move the optical system 13 and the supporter 22 while maintaining a positional relationship between the emission position and the measurement position so that the pulsed light 12 is emitted toward the high-resolution region 23. By doing so, a region in which a photoacoustic wave can be received with high sensitivity overlaps a region in which the amount of light is large, and therefore the signal intensity of a received signal is high and image noise in the region of interest can be considerably reduced in a single measurement.

The amount of light and the image noise level in each voxel in a region of interest when the object is irradiated with light vary in accordance with the shape of the object, the optical coefficients (such as the absorption coefficient, the scattering coefficient, the attenuation coefficient, and the like) of the object, the optical coefficients of the acoustic matching member, and the like. Therefore, the controller 19C may determine the emission position or the measurement position by using information based on the shape of the object. That is, on the basis of information about the shape of the object, the controller 19C may determine the emission position so that the total light amount due to light emission at a plurality of light emission timings corresponding to each voxel in the region of interest 32 is larger than or equal to a threshold. On the basis of information about the shape of the object, the controller 19C may determine the measurement position so that the level of image noise included in a reconstructed image is lower than or equal to a predetermined threshold.

The controller 19C may acquire information about the shape of the object, the optical coefficients (such as the absorption coefficient, the scattering coefficient, the attenuation coefficient, and the like) of the object, the optical coefficients of the acoustic matching member, and the like, and may read an irradiation position pattern or a measurement position pattern corresponding to the acquired information. The controller 19C controls the scanner 21 and the light source 11 so that emission positions and measurement positions form the emission position pattern or the measurement position pattern obtained in this way. For example, the controller 19C acquires information based on the shape of the object. Then, the controller 19C reads an emission position pattern or a measurement position pattern corresponding to the information based on the shape of the object from the storage unit 19B, in which a plurality of emission position patterns or measurement position patterns associated with the shape of the object have been stored.

When a tentative emission position pattern or a tentative measurement position pattern is assumed, the operation unit 19A estimates the amount of light or the image noise level in each voxel on the basis of parameters, such as the shape of the object, the optical coefficients of the object, and the optical coefficients of the acoustic matching member. Next, the operation unit 19A determines whether or not the amount of light in each voxel is larger than or equal to a predetermined threshold or whether or not the image noise in each voxel is lower than or equal to a predetermined threshold. If the amount of light in each voxel is larger than or equal to a predetermined threshold, the operation unit 19A transmits information about the emission position pattern assumed by the controller 19C. Then, the controller 19C controls the emission position by controlling the light irradiation unit on the basis of the information about the emission position pattern received from the operation unit 19A. If the image noise level in each voxel is lower than or equal to a predetermined threshold, the operation unit 19A transmits information about the measurement position pattern assumed by the controller 19C. Then, the controller 19C controls the measurement position by controlling the scanner on the basis of the information about the measurement position pattern received from the operation unit 19A. On the other hand, if the amount of light in each voxel is smaller than a predetermined threshold, the operation unit 19A assumes a new emission position pattern, estimates the amount of light in each voxel, and compares the amount of light with the threshold. If the image noise level in each voxel is higher than the predetermined threshold, the operation unit 19A assumes a new measurement position pattern, estimates the noise level in each voxel, and compares the image noise level with the threshold.

In the present invention, the term "information based on the shape of the object" refers to information about the position coordinates of the surface of the object or information about the type of the holding unit. The phrase "acquires information based on the shape of the object" means that the controller 19C receives information based on the shape of the object.

Hereinafter, an example of a method with which the controller 19C acquires the information based on the shape of the object will be described.

The controller 19C may acquire the information based on the shape of the object on the basis of image data of the object acquired by the image capturing device 50. First, the operation unit 19A reads image data of the object, which has been acquired by the image capturing device 50, from the storage unit 19B. Next, the operation unit 19A calculates the coordinate information about the surface of the object on the basis of the image data of the object, and outputs the coordinate information to the controller 19C. For example, the operation unit 19A may calculate the coordinate information about the surface of the object on the basis of a plurality of image data items by using a three-dimensional measurement technology, such as a stereo method. Then, the controller 19C receives the information about the position coordinates of the surface of the object, which has ben output from the operation unit 19A, and can acquire the information as information about the shape of the object.

Information about the position coordinates of the holding unit 40, which is known beforehand, may be stored in the storage unit 19B. Then, the controller 19C can read the information about the position coordinates of the holding unit 40 from the storage unit 19B, and can acquire the information as information about the position coordinates of the surface of the object.

A detector 42, which detects the type of the holding unit 40 attached to an attachment portion 41 and outputs information about the type of the holding unit 40 to the computer 19, may be provided. Then, the controller 19C can receive the information about the type of the holding unit 40, which has been output from the detector 42, and can acquire the information as the information based on the shape of the object. For example, a reader that reads an ID chip, which is mounted on the holding unit 40 and representing the type of the holding unit 40, may be used as the detector 42. By doing so, the information based on the shape of the object can be acquired without performing calculation.

A user may input the type of the holding unit 40 to be used by using the input unit 24, and the input unit 24 may output the input information to the computer 19. Then, the controller 19C may receive the information about the type of the holding unit 40, which has been output from the input unit 24, and may acquire the information as the information based on the shape of the object. By doing so, the information based on the shape of the object can be acquired without performing calculation.

If the type of the holding unit 40 does not change and it is not assumed that the size of the holding unit 40 does not change due to the apparatus specifications, the information based on the shape of the object used by the controller 19C may be constant.

The controller 19C can acquire the optical coefficients of the object or the optical coefficients of the acoustic matching member, which have been calculated by using a known method such as time-resolved spectroscopy. A user may input the information based on the shape of the object, the optical coefficients of the object, or the optical coefficients of the acoustic matching member by using the input unit 24; and the controller 19C may receive such information items.

## INFORMATION ACQUISITION APPARATUS

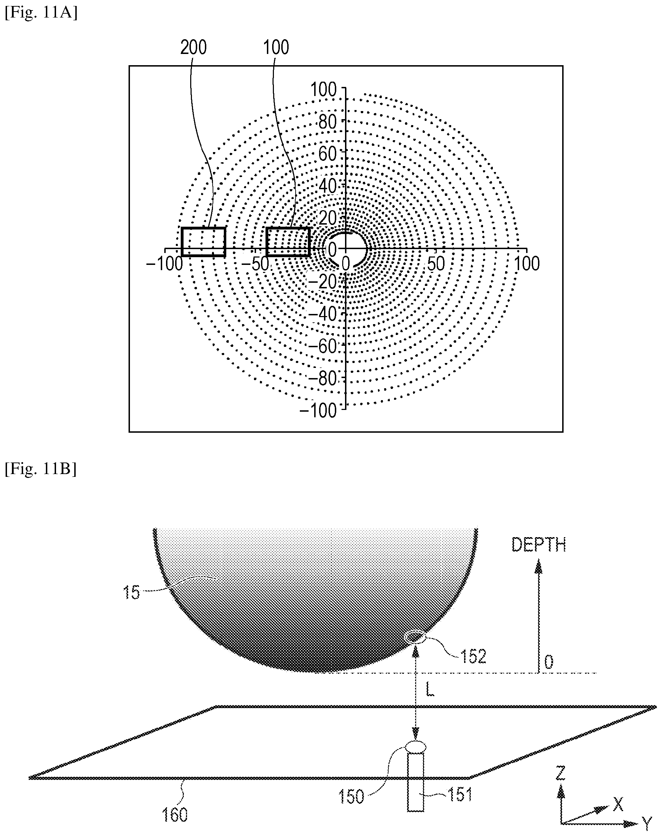

Next, referring to Figs. 11A and 11B, an information acquisition apparatus for acquiring information about an object by using a photoacoustic wave generated by irradiating the object with light will be described.

The information acquisition apparatus includes the aforementioned light irradiation unit, a scanning unit, a holding unit, a receiving unit, and a processing unit.

The light irradiation unit (denoted by 151 in Fig. 11B), for irradiating the object with light, has, for example, a light emission end for emitting light that is guided from the aforementioned light source through an optical waveguide.

For example, the light irradiation unit is structured so that the light irradiation unit can periodically emit pulsed light.

The scanning unit moves an emission position 150 of light, from which light is emitted from the light irradiation unit 151, relative the object in an in-plane direction (such as a direction parallel to the X-Y plane shown in Fig. 11B). Needless to say, the scanning unit may move the object, the light irradiation unit 151, or both of these. In a case where the object is a breast or the like, in consideration of a load on a subject, the light irradiation unit 151 may be moved.

The holding unit holds the object. To be specific, the holding unit holds the object so that the object has a portion in which the distance L from the emission position 150 of the light irradiation unit to a light irradiation position 152 on a surface of the object differs in accordance with the emission position 150. Here, the distance L is a length in a direction perpendicular to the in-plane direction.

The holding unit has a concave shape, such as a hemispherical shape, for holding a breast, which is an example of the object. As long as the holding unit has an opening for inserting a breast or the like therethrough, it is not necessary that the holding unit have a structure that supports the breast or the like in a direction (-Z direction) opposite to the direction of gravity.

The emission position 150 of light is a position from which light is emitted from the light irradiation unit. The light irradiation position 152 is a position on the object surface irradiated with light.

The dots in Fig. 11A show an example of a movement path of the emission position 150 of light. The intensity of light emitted from the emission position may be controlled so as to be constant between the dots. When emitting light beams having two wavelengths ($\lambda 1$, $\lambda 2$), the light beams may be alternately omitted, that is, in the order of $\lambda 1$, $\lambda 2$, $\lambda 1$, $\lambda 2$, $\lambda 1$ .... The pulsed light beams having the two wavelengths $\lambda 1$ and $\lambda 2$ may be emitted from one emission position.

The receiving unit, which is a unit for receiving a photoacoustic wave, includes, for example, capacitive micro-machined ultrasonic transducers (CMUTs).

As described above, the processing unit acquires information about the object by using a signal output from the receiving unit.

The receiving unit may be structured so that the receiving unit can be moved by the scanning unit in synchronism with the light irradiation unit. For example, a support member provided with the light irradiation unit and the receiving unit is prepared, and the support member is moved relative to the object.

A plurality of emission positions formed by the scanning unit at least include a first emission position group (for example, emission positions in a region 100 of Fig. 11A) and a second emission position group (for example, emission positions in a region 200 of Fig. 11A).

The first emission position group includes a plurality of emission positions for each of which the distance from the emission position 150 to the light irradiation position 152 is in a first range.

Likewise, the second emission position group includes a plurality of emission positions for each of which the distance from the emission position 150 to the light irradiation position 152 is in a second range that is constituted by values larger than any value in the first range.

In Fig. 11A, a large number of emission positions are drawn in one region 100 (or 200). However, a region including at least several emission positions may be considered.

The distance between emission positions in one group may gradually change.

The scanning unit is controlled so that the density of the emission positions included in the first emission position group is higher than the density of the emission positions included in the second emission position group.

Here, the density of the emission positions is the density of the number of emission positions in a predetermined area, and may be also called the surface density. Note that, in a case where pulsed light is emitted twice at one emission position, the number of the emission position is counted as two.

In the present apparatus, the distribution of a plurality of emission positions in a predetermined area may differ in accordance with an in-plane direction in which the light irradiation unit moves.

The density of the distribution of emission positions may be controlled by maintaining the cyclic frequency of pulsed light to be constant and by modulating the scanning speed of the scanning unit. Alternatively, the density of the distribution of emission positions may be controlled by modulating the cyclic frequency of emitted pulsed light while maintaining the scanning speed of the scanning unit to be constant. Further alternatively, both of the modulation of the scanning speed and the modulation of the cyclic frequency of pulsed light may be used.

The scanning unit may perform scanning so that the plurality of emission positions form a spiral movement path. The information acquisition apparatus may be structured so that, at the plurality of emission positions, pulsed light beams having wavelengths that differ from each other are alternately emitted from the light irradiation unit.

The scanning unit may be controlled so that the distance between the plurality of emission positions that form the spiral movement path decreases from an outer part toward an inner part of the spiral movement path.

The light irradiation unit may have a plurality of emission ends, and the controller may move the emission positions by switching the emission end from which light is emitted.

**Other Embodiments**

The present invention may be carried out by performing a process described below. That is, software (program) implementing the functions of the embodiment described above is provided to a system or an apparatus through a network or various storage media, and a computer (or CPU, MPU, or the like) of the system or the apparatus reads the program and executes the process.

**Example 1**

An example of a photoacoustic apparatus that realizes photoacoustic imaging will be described. Here, an example of a case where the object is a human breast will be shown.

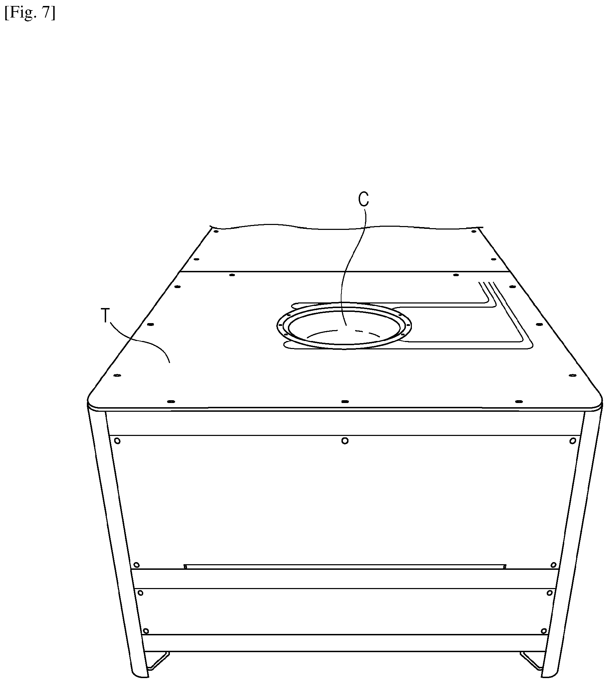

Fig. 7 illustrates a photoacoustic apparatus for measuring a breast. The apparatus includes a table (T), on which a subject lies, and a hemispherical plastic cup (C), which has a thickness of 0.02 inches. The hemispherical plastic cup (C), which corresponds to an object holding unit, is a breast cup for holding a breast. The breast cup (C) as an object holding unit has a curved holding surface and holds a breast in a shape along the curved holding surface. In order to keep acoustic matching between the breast and the breast cup (C), a small amount of water is poured into the breast cup.

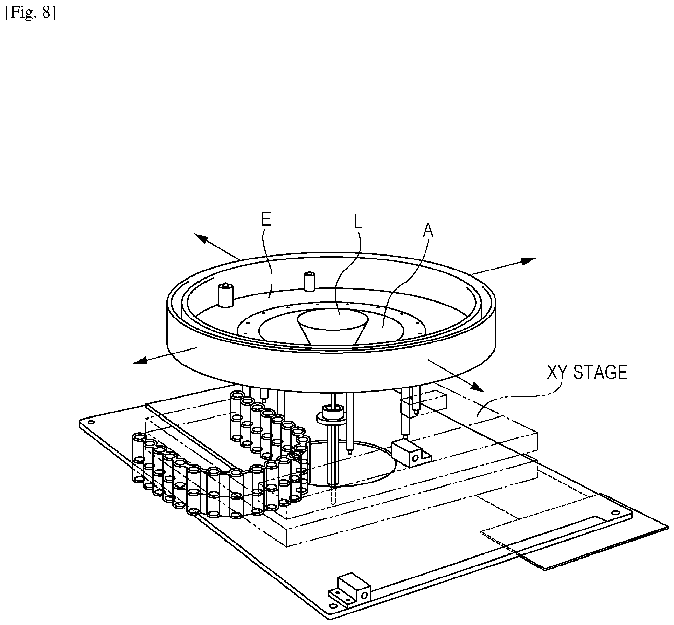

Fig. 8 illustrates a hemispherical array receiver (A), which is disposed below the breast cup, and a two-axis stage (XY), which moves the array receiver (A). The two-axis stage is controlled by the computer 19. The array receiver (A) includes a hemispherical supporter (having a radius of 127 mm), which is made of ABS plastic, and acoustic wave receiving elements of 512 ch. Each acoustic wave receiving element has a diameter of 3 mm width and is made of a piezoelectric material having a center frequency of 2 MHz.

The array receiver (A) and the space inside a plastic basin (E) for holding the array receiver (A) are filled with water for keeping acoustic matching of the acoustic wave receiving elements of 512 ch and the breast cup. The water has been filtered through a reverse osmosis membrane. A pulsed laser beam (75 ns, 300 mJ/pulse) emitted from an alexandrite laser has a width of 7 mm, and the object is irradiated with the laser beam through a movable arm. The movable arm moves together with the array receiver (A), and the laser beam (L) is emitted perpendicularly from a bottom surface of a probe. The diameter of the laser beam is increased by using a convex lens having a diameter of 12 mm, which is disposed at a bottom surface of the array receiver (A), and finally the bottom surface of the breast cup (C) is irradiated with a diameter of 60 mm. The peak intensity of the amount of light at the center is approximately 10 mJ/cm2, which is lower than the MPE recommended by ANSI.

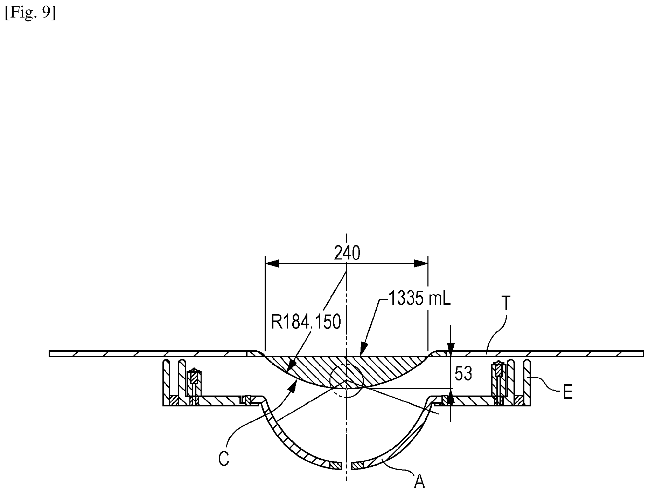

Fig. 9 is a schematic sectional view of the photoacoustic apparatus, representing the relationship among the array receiver (A), the basin (E), the table (T), and the breast cup (C). The basin (E) holds the array receiver (A) so that the array receiver (A) can move parallelly along a breast surface. The basin (E) is configured to be filled with water so as to maintain acoustic matching of the array receiver and the breast. The maximum imaging volume (1335 mL) is determined in accordance with the radius of curvature of the breast cup (C), the width (184 mm) of the opening in which the breast is to be placed, and the depth of the cup. The maximum imaging volume is shown in Fig. 9.

Regarding this system, by performing simulation with consideration of the apparatus characteristics, image noise with which a red dot pattern in a contrast phantom can be recognized with a contrast 5 was calculated for each voxel. In the present example, the distribution shown in Fig. 5A was used as the distribution of emission positions and the measurement position that satisfies the image noise. A spiral movement path was used as the movement path. As a comparative example, the measurement position and the emission position were sampled at a substantially equal distance as shown in Fig. 4A. In the present example and the comparative example, the number of the measurement positions and the emission positions were 2048, and the maximum radius of movement of the scanner 21 was 100 mm. Data from the acoustic wave receiving elements of 512 ch for each light emission was digitally converted with 20 MHzd, 2048 sampling, and 12 bit. Subsequently, on the basis of the received signal data, a three-dimensional photoacoustic image was reconstructed by using a backprojection algorithm.

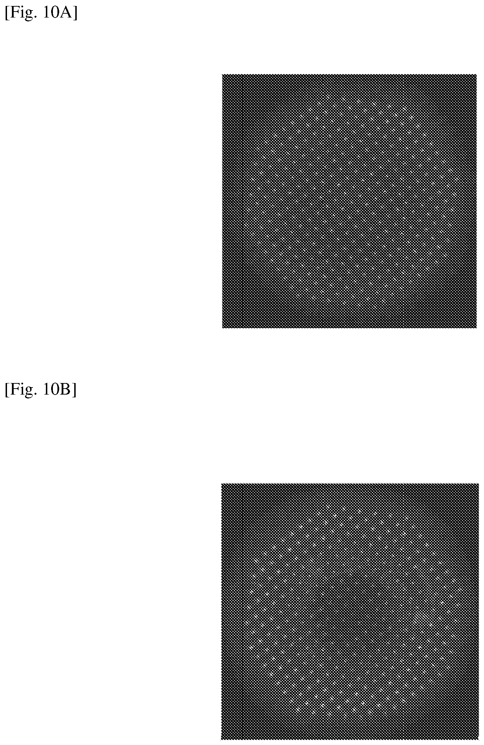

Next, the result of examining the effect of the present example by using the contrast phantom will be described. The contrast phantom was a region of 20 cm in which red dots were dispersed, and the contrast phantom was imaged. The red dot array was buried at a depth of 30 mm in a polyurethane resin and was located at the center of the breast cup (C). Figs. 10A and 10B show photoacoustic images of the contrast phantom at a thickness of about 30 mm, which were acquired at the emission positions and the measurement positions according to present example, which are shown in Fig. 5A, and the emission positions and the measurement positions according to the comparative example, which are shown in Fig. 4A. That is, Fig. 10A illustrates a photoacoustic image captured by using a spiral scan pattern in which the density of the distribution of the measurement positions and the emission positions increases toward the center of the distribution. On the other hand, Fig. 10B illustrates a photoacoustic image captured by using a spiral scan pattern in which the density of the distribution of the measurement positions and the emission positions is substantially uniform.

By comparing Fig. 10A with Fig. 10B, all dot patterns can be recognized with the same brilliance in Fig. 10A. On the other hand, it is difficult to recognize dot patterns in a central part of the image of Fig. 10B. The reason for this is as follows. In the photoacoustic apparatus according to the present example, the breast cup has a hemispherical shape as shown in Fig. 9. Therefore, a phantom disposed in the breast cup has a large thickness at a central part and a small thickness decreases at a peripheral part. When such a phantom is irradiated with a laser beam from below as illustrated in Fig. 1, the amount of light at the central part is low when a plane at the same thickness is compared, because the central part of the phantom is thick. As a result, the generated acoustic pressure generated from the red dots, which are the contrast phantom, is lower at the central part and higher in the peripheral part. That is, due to the light amount distribution in the phantom, the generated acoustic pressure differs between the dots, which have the same absorption coefficient. Therefore, when an image having a uniform image noise is formed, as shown in Fig. 10B, an image in which the brilliance of the dots at the central part is low as shown in Fig. 10B is formed. On the other hand, a case where the density of the distribution of the measurement positions and the emission position is increased toward the center in order to further reduce image noise in the central part, in which the generated acoustic pressure is low, is considered. In this case, in a region in which the density of the distribution of the measurement positions and the emission positions is high, image noise due to image reconstruction artifacts and image noise due to system noise are reduced. Therefore, when an image is formed by image reconstruction, an image in which the red dots are uniformly displayed as in Fig. 10A, is formed. That is, when the brilliance of the red dots is denoted by S and the brilliance of a region surrounding the red dots is denoted by N, the image shown in Fig. 10A can maintain a substantially uniform S/N (image contrast ratio) at the same depth.

As heretofore described, with the photoacoustic apparatus according to the present example, the level of image noise included in each voxel of a region of interest can be reduced to a level lower than or equal to a threshold.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

**Claims**

1. A photoacoustic apparatus comprising:

   a light irradiation unit that irradiates an object with light;
   an acoustic wave receiving unit that receives an acoustic wave generated by irradiating the object with light emitted from the light irradiation unit and that outputs an electric signal;
   a control unit that controls the light irradiation unit; and
   a processing unit that processes the electric signal, wherein the control unit controls the light irradiation unit so that a density of a distribution of emission positions in a plurality of times of light emission by the light irradiation unit is higher in an inner part of the distribution of the emission positions than in an outer part of the distribution,

   wherein the acoustic wave receiving unit outputs a plurality of electric signals corresponding to the plurality of times of light emission by receiving acoustic waves generated by the plurality of times of light emission, and
   wherein the processing unit acquires image data on the basis of the plurality of electric signals.

2. The photoacoustic apparatus according to Claim 1, wherein the light irradiation unit includes a light source that emits light, an optical system that irradiates the object with light from the light source, and an emission position moving unit that moves relative positions of the object and the optical system, and
   wherein the control unit controls driving of the emission position moving unit.

3. The photoacoustic apparatus according to Claim 2, wherein the light source emits light with a predetermined cyclic frequency.

4. The photoacoustic apparatus according to Claim 2 or 3,
   wherein the control unit controls the emission position moving unit so that a path of movement of the optical system due to the emission position moving unit is spiral.

5. The photoacoustic apparatus according to Claim 1, wherein the light irradiation unit includes a light source that emits light and an optical system that irradiates the object with light from the light source, wherein the optical system includes a plurality of emission ends, and
   wherein the control unit performs control so that one of the plurality of emission ends from which light is emitted differs between first light emission and second light emission of the plurality of times of light emission.

6. The photoacoustic apparatus according to any one of Claims 1 to 5, further comprising:

   a measurement position moving unit that moves relative positions of the object and the acoustic wave receiving unit,
   wherein the acoustic wave receiving unit includes a plurality of acoustic wave receiving elements that are disposed so that directional axes of the plurality of acoustic wave receiving elements converge, and
   wherein the control unit controls driving of the light irradiation unit and the measurement position moving unit so that light from the light irradiation unit is emitted toward a region in which the directional axes converge in the plurality of times of light emission.

7. The photoacoustic apparatus according to any one of Claims 1 to 6,
   wherein the control unit controls the light irradiation unit so that the number of times of light emission differs between the emission positions.

8. A photoacoustic apparatus comprising:

   a light irradiation unit that irradiates an object with light;
   an acoustic wave receiving unit that receives an acoustic wave generated by irradiating the object with the light from the light irradiation unit and that outputs an electric signal;
   a measurement position moving unit that moves relative positions of the object and the acoustic wave receiving unit;
   a control unit that controls driving of the measurement position moving unit; and
   a processing unit that processes the electric signal,

   wherein the control unit controls the measurement position moving unit so that a density of a distribution of measurement positions of the acoustic wave receiving unit in a plurality of times of light emission by the light irradiation unit is higher in an inner part of the distribution of the measurement positions than in an outer part of the distribution,
wherein the acoustic wave receiving unit outputs a plurality of electric signals corresponding to the plurality of times of light emission by receiving acoustic waves generated by the plurality of times of light emission, and
wherein the processing unit acquires image data on the basis of the plurality of electric signals.

9. The photoacoustic apparatus according to Claim 8,
wherein the acoustic wave receiving unit includes a plurality of acoustic wave receiving elements that are disposed so that directional axes of the plurality of acoustic wave receiving elements converge.

10. The photoacoustic apparatus according to Claim 8 or 9,
wherein the control unit controls the measurement position moving unit so that a path of movement of the acoustic wave receiving unit due to the measurement position moving unit is spiral.

11. The photoacoustic apparatus according to any one of Claims 8 to 10,
wherein the acoustic wave receiving unit includes a plurality of acoustic wave receiving elements that are disposed so that directional axes of the plurality of acoustic wave receiving elements converge, and
wherein the control unit controls driving of the light irradiation unit and the measurement position moving unit so that light from the light irradiation unit is emitted toward a region in which the directional axes converge in the plurality of times of light emission.

12. The photoacoustic apparatus according to any one of Claims 8 to 10,
wherein the control unit controls the moving unit so that the number of times of light emission differs between the measurement positions.

13. The photoacoustic apparatus according to any one of Claims 1 to 12, further comprising:

a holding unit that has a curved holding surface and that holds the object.

14. An information acquisition apparatus for acquiring information about an object by using a photoacoustic wave generated by irradiating the object with light, the information acquisition apparatus comprising:

a light irradiation unit for irradiating the object with light;

a scanning unit that moves an emission position, from which light is emitted from the light irradiation unit, relative to the object in an in-plane direction;
a holding unit that holds the object so that the object has a portion in which a distance from the emission position of the light irradiation unit to a light irradiation position on a surface of the object differs in accordance with the emission position;
a receiving unit for receiving the photoacoustic wave; and
a processing unit for acquiring the information about the object by using a signal output from the receiving unit,
wherein the emission position formed by the scanning unit at least includes

a first emission position group including a plurality of the emission positions for each of which the distance from the emission position to the light irradiation position is in a first range, and
a second emission position group including a plurality of the emission positions for each of which the distance from the emission position to the light irradiation position is in a second range that is constituted by values larger than any value in the first range, and

wherein the scanning unit is controlled so that a density of the emission positions included in the first emission position group is higher than the density of the emission positions included in the second emission position group.

15. The information acquisition apparatus according to Claim 14,
wherein the light irradiation unit periodically emits pulsed light.

16. The information acquisition apparatus according to Claim 14 or 15,
wherein the holding unit has a concave shape for holding the object.

17. The information acquisition apparatus according to any one of Claims 14 to 16,
wherein the receiving unit is moved by the scanning unit in synchronism with the light irradiation unit.

18. The information acquisition apparatus according to any one of Claims 14 to 17,
wherein the scanning unit performs scanning so that the plurality of emission positions form a spiral movement path.

19. The information acquisition apparatus according to Claim 18,

wherein the scanning unit is controlled so that a distance between the plurality of emission positions that form the spiral movement path decreases from an outer part toward an inner part of the spiral movement path.

20. The information acquisition apparatus according to Claim 18 or 19,
wherein, at the plurality of emission positions, pulsed light beams having wavelengths that differ from each other are alternately emitted from the light irradiation unit.

**Patentansprüche**

1. Fotoakustische Vorrichtung, umfassend:

   eine Lichtbestrahlungseinheit, die ein Objekt mit Licht bestrahlt;
   eine Akustikwellenempfangseinheit, die eine Akustikwelle empfängt, welche durch Bestrahlung des Objekts mit aus der Lichtbestrahlungseinheit emittiertem Licht erzeugt wird, und ein elektrisches Signal ausgibt;
   eine Steuereinheit, die die Lichtbestrahlungseinheit steuert; und
   eine Verarbeitungseinheit, die das elektrische Signal verarbeitet,
   wobei die Steuereinheit die Lichtbestrahlungseinheit derart steuert, dass eine Emissionspositionsverteilungsdichte in mehreren Lichtemissionszeitpunkten durch die Lichtbestrahlungseinheit in einem inneren Teil der Emissionspositionsverteilung höher ist als in einem äußeren Teil der Verteilung,
   wobei die Akustikwellenempfangseinheit durch Empfang von durch die Lichtemission zu mehreren Lichtemissionszeitpunkten erzeugten Akustikwellen mehrere den mehreren Lichtemissionszeitpunkten entsprechende elektrische Signale ausgibt, und
   wobei die Verarbeitungseinheit Bilddaten basierend auf den mehreren elektrischen Signalen erfasst.

2. Fotoakustische Vorrichtung nach Anspruch 1,

   wobei die Lichtbestrahlungseinheit eine Lichtquelle enthält, die Licht ausgibt, ein optisches System, das das Objekt mit Licht aus der Lichtquelle bestrahlt sowie eine Emissionspositionsbewegungseinheit, die Relativpositionen des Objekts und des optischen Systems bewegt, und
   wobei die Steuereinheit das Antreiben der Emissionspositionsbewegungseinheit steuert.

3. Fotoakustische Vorrichtung nach Anspruch 2, wobei die Lichtquelle Licht mit einer vorbestimmten zyklischen Frequenz emittiert.

4. Fotoakustische Vorrichtung nach einem der Ansprüche 2 oder 3,

   wobei die Steuereinheit die Emissionspositionsbewegungseinheit derart steuert, dass ein Bewegungspfad des optischen Systems durch die Emissionspositionsbewegungseinheit spiralförmig ist.

5. Fotoakustische Vorrichtung nach Anspruch 1,

   wobei die Lichtbestrahlungseinheit eine Lichtquelle enthält, die Licht emittiert sowie ein optisches System, das das Objekt mit Licht aus der Lichtquelle bestrahlt,
   wobei das optische System mehrere Emissionsendbereiche enthält, und
   wobei die Steuereinheit eine Steuerung derart durchführt, dass einer der mehreren Emissionsendbereiche, aus denen Licht emittiert wird, sich zwischen der ersten Lichtemission und der zweiten Lichtemission der mehreren Lichtemissionszeitpunkte unterscheidet.

6. Fotoakustische Vorrichtung nach einem der Ansprüche 1 bis 5, ferner umfassend:

   eine Messpositionsbewegungseinheit, die Relativpositionen des Objekts und der Akustikwellenempfangseinheit bewegt,
   wobei die Akustikwellenempfangseinheit mehrere Akustikwellenempfangselemente enthält, die derart angeordnet sind, dass Richtungsachsen der mehreren Akustikwellenempfangselemente zusammenlaufen, und
   wobei die Steuereinheit das Antreiben der Lichtbestrahlungseinheit und der Messpositionsbewegungseinheit derart steuert, dass Licht aus der Lichtbestrahlungseinheit in Richtung eines Gebiets emittiert wird, in dem die Richtungsachsen in den mehreren Lichtemissionszeitpunkten zusammenlaufen.

7. Fotoakustische Vorrichtung nach einem der Ansprüche 1 bis 6, wobei die Steuereinheit die Lichtbestrahlungseinheit derart steuert, dass sich die Anzahl der Lichtemissionszeitpunkte zwischen den Emissionspositionen unterscheidet.

8. Fotoakustische Vorrichtung, umfassend:

   eine Lichtbestrahlungseinheit, die ein Objekt mit Licht bestrahlt;
   eine Akustikwellenempfangseinheit, die eine Akustikwelle empfängt, welche durch Bestrahlung des Objekts mit Licht aus der Lichtbestrahlungseinheit erzeugt wird, und ein elektrisches Signal ausgibt;

eine Messpositionsbewegungseinheit, die Relativpositionen des Objekts und der Akustikwellenempfangseinheit bewegt;

eine Steuereinheit, die das Antreiben der Messpositionsbewegungseinheit steuert; und

eine Verarbeitungseinheit, die das elektrische Signal verarbeitet,

wobei die Steuereinheit die Messpositionsbewegungseinheit derart steuert, dass eine Messpositionsverteilungsdichte der Akustikwellenempfangseinheit in mehreren Lichtemissionszeitpunkten durch die Lichtbestrahlungseinheit in einem inneren Teil der Messpositionsverteilung höher ist als in einem äußeren Teil der Verteilung,

wobei die Akustikwellenempfangseinheit durch Empfang von durch die Lichtemission zu mehreren Lichtemissionszeitpunkten erzeugten Akustikwellen mehrere den mehreren Lichtemissionszeitpunkten entsprechende elektrische Signale ausgibt, und

wobei die Verarbeitungseinheit Bilddaten basierend auf den mehreren elektrischen Signalen erfasst.

9. Fotoakustische Vorrichtung nach Anspruch 8, wobei die Akustikwellenempfangseinheit mehrere Akustikwellenempfangselemente enthält, die derart angeordnet sind, dass Richtungsachsen der mehreren Akustikwellenempfangselemente zusammenlaufen.

10. Fotoakustische Vorrichtung nach einem der Ansprüche 8 oder 9,

wobei die Steuereinheit die Messpositionsbewegungseinheit derart steuert, dass ein Bewegungspfad der Akustikwellenempfangseinheit durch die Messpositionsbewegungseinheit spiralförmig ist.

11. Fotoakustische Vorrichtung nach einem der Ansprüche 8 bis 10,

wobei die Akustikwellenempfangseinheit mehrere Akustikwellenempfangselemente enthält, die derart angeordnet sind, dass Richtungsachsen der mehreren Akustikwellenempfangselemente zusammenlaufen, und

wobei die Steuereinheit das Antreiben der Lichtbestrahlungseinheit und der Messpositionsbewegungseinheit derart steuert, dass Licht aus der Lichtbestrahlungseinheit in Richtung eines Gebiets emittiert wird, in dem die Richtungsachsen in den mehreren Lichtemissionszeitpunkten zusammenlaufen.

12. Fotoakustische Vorrichtung nach einem der Ansprüche 8 bis 10,

wobei die Steuereinheit die Bewegungseinheit derart steuert, dass sich die Anzahl der Lichtemissionszeitpunkte zwischen den Messpositionen unterscheidet.

13. Fotoakustische Vorrichtung nach einem der Ansprüche 1 bis 12, ferner umfassend:

eine Halteeinheit, die eine gekrümmte Halteoberfläche aufweist und die das Objekt hält.

14. Informationserfassungsvorrichtung zum Erfassen von Information über ein Objekt durch Verwenden einer durch Bestrahlen des Objekts mit Licht erzeugten Fotoakustikwelle, wobei die Informationserfassungsvorrichtung umfasst:

eine Lichtbestrahlungseinheit zum Bestrahlen des Objekts mit Licht;

eine Abtasteinheit, die eine Emissionsposition bewegt, aus der Licht von der Lichtbestrahlungseinheit emittiert wird, relativ zum Objekt in einer Richtung der Ebene;

eine Halteeinheit, die das Objekt derart hält, dass das Objekt einen Abschnitt aufweist, in dem sich eine Entfernung von der Emissionsposition der Lichtbestrahlungseinheit zu einer Lichtbestrahlungsposition auf einer Oberfläche des Objekts gemäß der Emissionsposition unterscheidet;

eine Empfangseinheit zum Empfangen der fotoakustischen Welle; und

eine Verarbeitungseinheit zum Erfassen der Information über das Objekt durch Verwenden eines aus der Empfangseinheit ausgegebenen Signals,

wobei die durch die Abtasteinheit gebildete Emissionsposition zumindest enthält:

eine erste Emissionspositionsgruppe, enthaltend mehrere der Emissionspositionen, für die jeweils die Entfernung von der Emissionsposition zur Lichtbestrahlungsposition in einem ersten Gebiet liegt, und

eine zweite Emissionspositionsgruppe, enthaltend mehrere der Emissionspositionen, für die jeweils die Entfernung von der Emissionsposition zur Lichtbestrahlungsposition in einem zweiten Gebiet liegt, das durch Werte gebildet ist, die größer als ein beliebiger Wert im ersten Gebiet sind, und

wobei die Abtasteinheit derart gesteuert wird, dass eine Dichte der Emissionspositionen, die in der ersten Emissionspositionsgruppe enthalten sind, höher ist als eine Dichte der Emissionspositionen, die in der zweiten Emissionspositionsgruppe enthalten sind.

15. Informationserfassungsvorrichtung nach Anspruch 14,

   wobei die Lichtbestrahlungseinheit pulsierendes Licht periodisch emittiert.

16. Informationserfassungsvorrichtung nach Anspruch 14 oder 15,

   wobei die Halteeinheit eine konkave Form zum Halten des Objekts aufweist.

17. Informationserfassungsvorrichtung nach einem der Ansprüche 14 bis 16, wobei die Empfangseinheit durch die Abtasteinheit synchron zur Lichtbestrahlungseinheit bewegt wird.

18. Informationserfassungsvorrichtung nach einem der Ansprüche 14 bis 17,

   wobei die Abtasteinheit das Abtasten derart durchführt, dass die mehreren Emissionspositionen einen spiralförmigen Bewegungspfad bilden.

19. Informationserfassungsvorrichtung nach Anspruch 18,

   wobei die Abtasteinheit derart gesteuert wird, dass eine Entfernung zwischen den mehreren Emissionspositionen, die den spiralförmigen Bewegungspfad bilden, von einem äußeren Teil in Richtung einem inneren Teil des spiralförmigen Bewegungspfads abnimmt.

20. Informationserfassungsvorrichtung nach Anspruch 18 oder 19,

   wobei an den mehreren Emissionspositionen pulsierende Lichtstrahlen mit Wellenlängen, die sich voneinander unterscheiden, abwechselnd von der Lichtbestrahlungseinheit emittiert werden.

## Revendications

1. Appareil photo-acoustique, comprenant :

   une unité d'exposition à un rayonnement de lumière qui expose un objet à un rayonnement de lumière ;
   une unité de réception d'onde acoustique qui reçoit une onde acoustique générée par une exposition de l'objet à un rayonnement de lumière émis à partir de l'unité d'exposition à un rayonnement de lumière et qui délivre un signal électrique ;
   une unité de commande qui commande l'unité d'exposition à un rayonnement de lumière ; et
   une unité de traitement qui traite le signal électrique,

   dans lequel l'unité de commande commande l'unité d'exposition à un rayonnement de lumière de sorte qu'une densité d'une distribution de positions d'émission d'une pluralité d'émissions de lumière par l'unité d'exposition à un rayonnement de lumière soit supérieure dans une partie interne de la distribution de positions d'émission que dans une partie externe de la distribution,
   dans lequel l'unité de réception d'onde acoustique délivre une pluralité de signaux électriques correspondant à la pluralité d'émissions de lumière par une réception d'ondes acoustiques générées par la pluralité d'émissions de lumière, et
   dans lequel l'unité de traitement acquiert des données d'image sur la base de la pluralité de signaux électriques.

2. Appareil photo-acoustique selon la revendication 1, dans lequel l'unité d'exposition à un rayonnement de lumière comprend une source de lumière qui émet de la lumière, un système optique qui expose l'objet à un rayonnement de lumière provenant de la source de lumière, et une unité de déplacement de positions d'émission qui déplace des positions relatives de l'objet et du système optique, et
   dans lequel l'unité de commande commande un pilotage de l'unité de déplacement de positions d'émission.

3. Appareil photo-acoustique selon la revendication 2, dans lequel la source de lumière émet de la lumière à une fréquence cyclique prédéterminée.

4. Appareil photo-acoustique selon la revendication 2 ou la revendication 3,
   dans lequel l'unité de commande commande l'unité de déplacement de positions d'émission de sorte qu'un trajet de déplacement du système optique dû à l'unité de déplacement de positions d'émission soit une spirale.

5. Appareil photo-acoustique selon la revendication 1, dans lequel l'unité d'exposition à un rayonnement de lumière comprend une source de lumière qui émet de la lumière et un système optique qui expose l'objet à de la lumière provenant de la source de lumière,
dans lequel le système optique comprend une pluralité d'extrémités d'émission, et
dans lequel l'unité de commande exécute une commande de sorte qu'une extrémité de la pluralité d'extrémités d'émission à partir de laquelle est émise de la lumière diffère entre une première émission de lumière et une seconde émission de lumière de la pluralité d'émissions de lumière.

6. Appareil photo-acoustique selon l'une quelconque des revendications 1 à 5, comprenant en outre :

une unité de déplacement de positions de mesure qui déplace des positions relatives de l'objet et de l'unité de réception d'onde acoustique, dans lequel l'unité de réception d'onde acoustique comprend une pluralité d'éléments de réception d'onde acoustique qui sont disposés de sorte que des axes directionnels de la pluralité d'éléments de réception d'onde acoustique convergent, et
dans lequel l'unité de commande commande un pilotage de l'unité d'exposition à un rayonnement de lumière et de l'unité de déplacement de positions de mesure de sorte que de la lumière provenant de l'unité d'exposition à un rayonnement de lumière soit émise en direction d'une région dans laquelle convergent les axes directionnels de la pluralité d'émissions de lumière.

7. Appareil photo-acoustique selon l'une quelconque des revendications 1 à 6,
dans lequel l'unité de commande commande l'unité d'exposition à un rayonnement de lumière de sorte que le nombre d'émissions de lumière diffère entre les positions d'émission.

8. Appareil photo-acoustique, comprenant :

une unité d'exposition à un rayonnement de lumière qui expose un objet à un rayonnement de lumière ;
une unité de réception d'onde acoustique qui reçoit une onde acoustique générée par une exposition de l'objet à un rayonnement de la lumière provenant de l'unité d'exposition à un rayonnement de lumière et qui délivre un signal électrique ;
une unité de déplacement de positions de mesure qui déplace des positions relatives de l'objet et de l'unité de réception d'onde acoustique ;
une unité de commande qui commande un pilotage de l'unité de déplacement de positions de mesure ; et
une unité de traitement qui traite le signal électrique,

dans lequel l'unité de commande commande l'unité de déplacement de positions de mesure de sorte qu'une densité d'une distribution de positions de mesure de l'unité de réception d'onde acoustique d'une pluralité d'émissions de lumière par l'unité d'exposition à un rayonnement de lumière soit supérieure dans une partie interne de la distribution des positions de mesure que dans une partie externe de la distribution,
dans lequel l'unité de réception d'onde acoustique délivre une pluralité de signaux électriques correspondant à la pluralité d'émissions de lumière par une réception d'ondes acoustiques générées par la pluralité d'émissions de lumière, et
dans lequel l'unité de traitement acquiert des données d'image sur la base de la pluralité de signaux électriques.

9. Appareil photo-acoustique selon la revendication 8, dans lequel l'unité de réception d'onde acoustique comprend une pluralité d'éléments de réception d'onde acoustique qui sont disposés de sorte que des axes directionnels de la pluralité d'éléments de réception d'onde acoustique convergent.

10. Appareil photo-acoustique selon la revendication 8 ou la revendication 9,
dans lequel l'unité de commande commande l'unité de déplacement de positions de mesure de sorte qu'un trajet de déplacement de l'unité de réception d'onde acoustique dû à l'unité de déplacement de positions de mesure soit une spirale.

11. Appareil photo-acoustique selon l'une quelconque des revendications 8 à 10,
dans lequel l'unité de réception d'onde acoustique comprend une pluralité d'éléments de réception d'onde acoustique qui sont disposés de sorte que des axes directionnels de la pluralité d'éléments de réception d'onde acoustique convergent, et
dans lequel l'unité de commande commande un pilotage de l'unité d'exposition à un rayonnement de lumière et de l'unité de déplacement de positions de mesure de sorte que de la lumière provenant de l'unité d'exposition à un rayonnement de lumière soit émise en direction d'une région dans laquelle convergent les axes directionnels de la pluralité d'émissions de lumière.

12. Appareil photo-acoustique selon l'une quelconque des revendications 8 à 10,
dans lequel l'unité de commande commande l'unité de déplacement de sorte que le nombre d'émissions de lumière diffère entre les positions de mesure.

13. Appareil photo-acoustique selon l'une quelconque des revendications 1 à 12, comprenant en outre :

une unité de maintien qui comporte une surface de maintien incurvée et qui maintient l'objet.

14. Appareil d'acquisition d'informations destiné à acquérir des informations concernant un objet au moyen d'une onde photo-acoustique générée par une exposition de l'objet à un rayonnement de lumière, l'appareil d'acquisition d'informations comprenant :

une unité d'exposition à un rayonnement de lumière destinée à exposer l'objet à un rayonnement de lumière ;
une unité de balayage qui déplace une position d'émission, à partir de laquelle est émise de la lumière provenant de l'unité d'exposition à un rayonnement de lumière, par rapport à l'objet dans une direction dans le plan ;
une unité de maintien qui maintient l'objet de sorte que l'objet ait une partie dans laquelle une distance de la position d'émission de l'unité d'exposition à un rayonnement de lumière à une position d'exposition à un rayonnement de lumière sur une surface de l'objet diffère conformément à la position d'émission ;
une unité de réception destinée à recevoir l'onde photo-acoustique ; et
une unité de traitement destinée à acquérir les informations concernant l'objet au moyen d'un signal délivré à partir de l'unité de réception,
dans lequel la position d'émission formée par l'unité de balayage comprend au moins

un premier groupe de positions d'émission comprenant une pluralité des positions d'émission pour lesquelles, individuellement, la distance de la position d'émission à la position d'exposition à un rayonnement de lumière s'inscrit dans une première plage, et
un second groupe de positions d'émission comprenant une pluralité des positions d'émission pour lesquelles, individuellement, la distance de la position d'émission à la position d'exposition à un rayonnement de lumière s'inscrit dans une seconde plage qui est constituée par des valeurs supérieures à une valeur quelconque de la première plage, et

dans lequel l'unité de balayage est commandée de sorte qu'une densité des positions d'émission comprises dans le premier groupe de positions d'émission soit supérieure à la densité des positions d'émission comprises dans le second groupe de positions d'émission.

15. Appareil d'acquisition d'informations selon la revendication 14,
dans lequel l'unité d'exposition à un rayonnement de lumière émet périodiquement de la lumière pulsée.

16. Appareil d'acquisition d'informations selon la revendication 14 ou la revendication 15,
dans lequel l'unité de maintien a une forme concave pour maintenir l'objet.

17. Appareil d'acquisition d'informations selon l'une quelconque des revendications 14 à 16,
dans lequel l'unité de réception est déplacée par l'unité de balayage de manière synchrone avec l'unité d'exposition à un rayonnement de lumière.

18. Appareil d'acquisition d'informations selon l'une quelconque des revendications 14 à 17,
dans lequel l'unité de balayage exécute un balayage de sorte que la pluralité de positions d'émission forme un trajet de déplacement en spirale.

19. Appareil d'acquisition d'informations selon la revendication 18,
dans lequel l'unité de balayage est commandée de sorte qu'une distance entre la pluralité de positions d'émission formant le trajet de déplacement en spirale aille en diminuant d'une partie externe en direction d'une partie interne du trajet de déplacement en spirale.

20. Appareil d'acquisition d'informations selon la revendication 18 ou la revendication 19,
dans lequel, au niveau de la pluralité de positions d'émission, des faisceaux de lumière pulsée ayant des longueurs d'onde qui diffèrent les unes des autres sont émis en alternance à partir de l'unité d'exposition à un rayonnement de lumière.

[Fig. 1]

[Fig. 2A]

[Fig. 2B]

[Fig. 3A]

[Fig. 3B]

[Fig. 4A]

[Fig. 4B]

[Fig. 5A]

[Fig. 5B]

[Fig. 6]

[Fig. 7]

[Fig. 8]

[Fig. 9]

[Fig. 10A]

[Fig. 10B]

[Fig. 11A]

[Fig. 11B]

**EP 3 188 647 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5713356 A

**Non-patent literature cited in the description**

- **M. XU ; L. V. WANG.** Photoacoustic imaging in biomedicine. *REVIEW OF SCIENTIFIC INSTURUMENT,* 2006, vol. 77, 041101
- Dedicated 3D photoacoustic breast imaging. **KRUGER ROBERT A et al.** MEDICAL PHYSICS. AIP, 10 October 2013, vol. 40
- **ROBERT A. KRUGER ; CHERIE M. KUZMIAK ; RICHARD B. LAM ; DANIEL R. REINECKE ; STEPHEN P. DEL RIO ; DOREEN STEED.** Dedicated 3D Photoacoustic Breast imaging. *Medical Physics,* 2013, vol. 40, 113301